# EUROPEAN PATENT SPECIFICATION

(11) **EP 0 408 760 B1**
(45) Date of publication and mention of the grant of the patent: **15.03.1995**
(21) Application number: 90901659.4
(22) Date of filing: 04.01.1990
(51) Int. Cl.: C07C 259/04, C07D 311/58, C07D 313/08, C07D 335/06, C07D 337/08, A61K 31/335, A61K 31/38, A61K 31/16, A61K 31/27

(54) **NEW HYDROXYLAMINE DERIVATIVE**
HYDROXYLAMIN-DERIVATE
NOUVEAU DERIVE D'HYDROXYLAMINE

(30) Priority: 30.12.1988 GB 8830427
(43) Date of publication of application: 23.01.1991
(73) Proprietor: FUJISAWA PHARMACEUTICAL CO., LTD., Osaka-shi Osaka 541 (JP)
(72) Inventor: OKU, Teruo, Ibaraki 305 (JP); KAWAI, Yoshio 15-2-405, Umezono 2-chome, Ibaraki 305 (JP); YATABE, Takumi, Tsukuba-shi Ibaraki 305 (JP); HASHIMOTO, Masashi, Tsukuba-shi Ibaraki 305 (JP)
(74) Representative: Türk, Gille, Hrabal, Leifert
(86) International application number: JP9000003
(87) International publication number: WO9007494

(56) References cited:
- EP-A- 0 196 184
- EP-A- 0 292 699

## Description

This invention relates to new hydroxylamine derivatives and pharmaceutically acceptable salts thereof which possess an inhibitory activity against 5-lipoxygenase and are useful as a medicament.

### BACKGROUND ART

Some compounds having an inhibitory activity against 5-lipoxygenase have been known as described in Japanese Patent Application Publication No. 63-264456 and EP-A-0 292 699.

### DISCLOSURE OF INVENTION

This invention relates to new hydroxyalmine derivatives and pharmaceutically acceptable salts thereof which have an inhibitory activity against 5-lipoxygenase, to processes for the preparation thereof, to a pharmaceutical composition comprising the same and to a method for the treatment of allergy or inflammatory in human beings or animals, and more particularly to method for treatment of asthma, psoriasis, hepatitis, pancreatitis, arthritis, nephritis, inflammatory bowel disease, septic shock, arteriosclerosis, myocardial infarction, cerebral vasospasm, rhinitis, conjunctivitis, dermatitis, rheumatism, peptic ulcer, gout and the like.

One object of this invention is to provide new and useful hydroxyalamine derivatives and pharmaceutically acceptable salts thereof which possess an inhibitory activity against 5-lipoxygenase.

Another object of this invention is to provide processes for the preparation of said hydroxylamine derivatives and salts thereof.

A further object of this invention is to provide a pharmaceutical composition comprising, as an active ingredient, said hydroxyalmine derivatives and pharmaceutically acceptable salts thereof.

Still further object of this invention is to provide a therapeutical method for the treatment of allergy or inflammatory, and more particularly of asthma, psoriasis, hepatitis, pancreatitis, arthritis, nephritis, inflammatory bowel disease, septic shock, arteriosclerosis, myocardial infarction, cerebral vasospasm, rhinitis, conjunctivitis, dermatitis, rheumatism, peptic ulcer, gout and the like, using said hydroxyalmine derivatives and pharmaceutically acceptable salts thereof.

The object hydroxylamine derivatives of this invention are new and can be represented by the following general formula [I] : 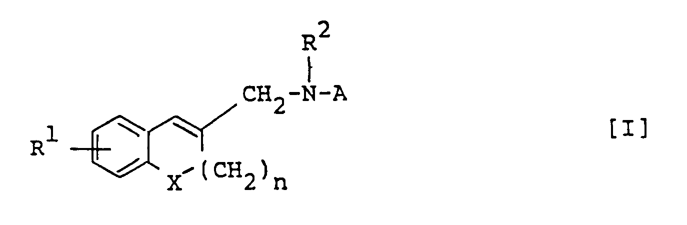
wherein
- R¹: is aryl, aryloxy, arylthio, ar(lower)alkyl or ar(lower)alkoxy,
- R²: is acyl,
- A: is hydroxy or protected hydroxy,
- X: is -CH₂-, -O- or -S-, and
- n: is an integer of 0 to 2,
with proviso that X is -CH₂- when n is 0.

The object compound [I] or its salt can be prepared by processes as illustrated in the following reaction schemes. 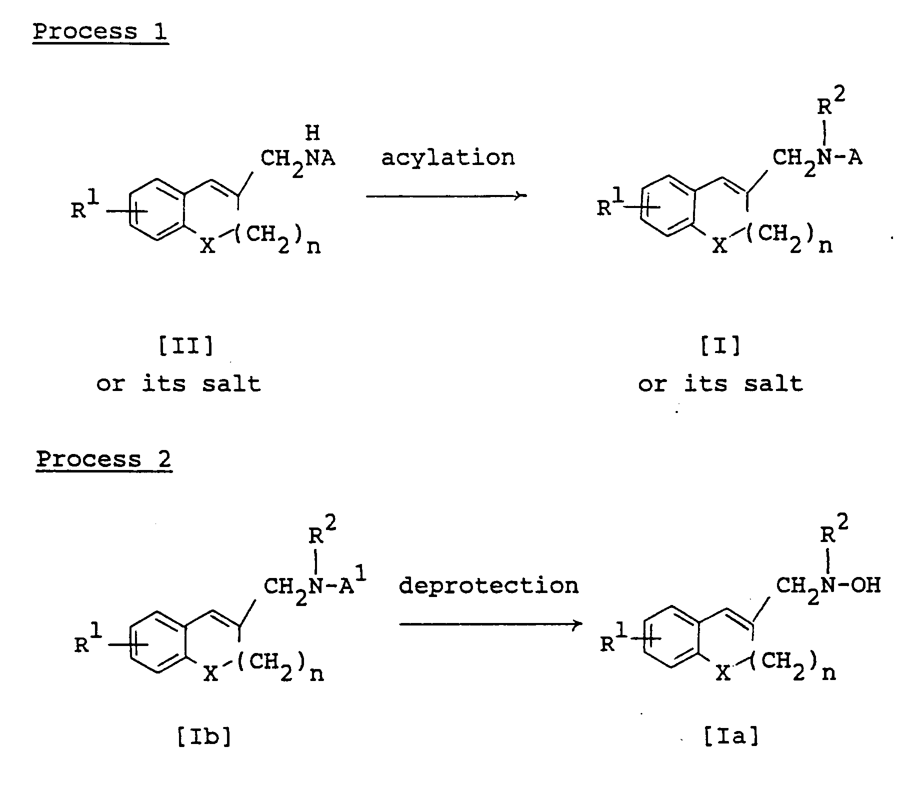
wherein
- R¹, R², X, A and n: are each as defined above, and
- A¹: is protected hydroxy.

In the above and subsequent description of the present specification, suitable examples of the various definitions to be included within the scope of the invention are explained in detail in the following.

The term "lower" is intended to mean a group having 1 to 6 carbon atom(s), unless otherwise provided.

Suitable "aryl" may be phenyl, naphthyl, tolyl, xylyl, mesityl, cumenyl, and the like, in which preferable one is phenyl.

Suitable "aryloxy" may be phenoxy, naphthyloxy, tolyloxy, xylyloxy, mesityloxy, cumenyloxy, and the like, in which preferable one is phenoxy.

Suitable "arylthio" may be phenylthio, naphthylthio, tolylthio, and the like, in which preferable one is phenylthio.

Suitable "ar(lower)alkyl" may be benzyl, phenethyl, phenylpropyl, benzhydryl, trityl, and the like, in which preferable one is benzyl.

Suitable "ar(lower)alkyloxy" may be benzyloxy, phenethyloxy, phenylpropyloxy, benzhydryloxy, trityloxy, and the like, in which preferable one is benzyloxy.

Suitable "acyl" may be carbamoyl, thiocarbamoyl, lower alkyl-carbamoyl (e.g. methylcarbamoyl, ethylcarbamoyl, etc.), lower alkyl-thiocarbamoyl (e.g. methylthiocarbamoyl, ethylthiocarbamoyl etc.), sulfamoyl, an aliphatic acyl, an aromatic acyl, a heterocyclic acyl, and the like, in which preferable one is carbamoyl, lower alkyl-carbamoyl, lower alkyl-thiocarbamoyl, an aliphatic acyl,etc.

The aliphatic acyl may be saturated or unsaturated, acyclic or cyclic ones, such as lower alkanoyl (e.g. formyl, acetyl, propionyl, butyryl, isobutyryl, valeryl, isovaleryl, pivaloyl, hexanoyl, etc.), lower alkanesulfonyl (e.g. mesyl, ethanesulfonyl, propanesulfonyl, etc.), lower alkoxycarbonyl (e.g. methoxycarbonyl, ethoxycarbonyl, propoxycarbonyl, isopropoxycarbonyl, butoxycarbonyl, tert-butoxycarbonyl, etc.), or the like.

Suitable hydroxy-protective group in the "protected hydroxy" may be a conventional one such as acyl mentioned above, substituted methyl (e.g. methoxymethyl, 2-methoxyethoxymethyl, etc.), tetrahydropyranyl, tetrahydrofuryl, substituted silyl (e.g. trimethylsilyl, etc.), or the like.

Suitable pharmaceutically acceptable salts of the object compounds [I] are conventional non-toxic salts and include an inorganic base salt such as an alkali metal salt [e.g. sodium salt potassium salt, etc.], an alkaline earth metal salt [e.g. calcium salt, magnesium salt, etc.] or an ammonium salt; an organic base salt such as an organic amine salt [e.g. methylamine salt, ethylamine salt, propylamine salt, isopropylamine salt, butylamine salt, tert-butylamine salt, dimethylamine salt, diethylamine salt, trimethylamine salt, triethylamine salt, pyridine salt, picoline salt, dicyclohexylamine salt, N,N'-dibenzylethylenediamine salt, etc.], a salt with an amino acid [e.g. arginine salt, aspartic acid salt, glutamic acid salt, etc.], and the like.

The processes for preparing the object compound [I] are explained in detail in the following.

### Process 1

The compound [I] or a salt thereof can be prepared by reacting a compound [II] or a salt with an acylating agent.

Suitable salt of the compound [II] may be salts with an organic acid (e.g. formic acid, acetic acid, propionic acid, etc.) and an inorganic acid (e.g. hydrochloric acid, hydrobromic acid, sulfuric acid, etc.).

The acylating agent may include an organic acid represented by the formula R²-OH, in which R² is acyl as illustrated above, or its reactive derivative or a salt thereof, a compound of the formula : R⁴-N=C=Y, wherein R⁴ is lower alkyl, tri(lower)alkylsilyl or trihalo(lower)alkanoyl and Y is =O or =S, and the like.

The suitable reactive derivative of the organic acid may be a conventional one such as an acid halide (e.g. acid chloride, acid bromide, etc.), an acid azide, an acid anhydride, an activated amide, an activated ester, etc.

Suitable "lower alkyl" may be methyl, ethyl, propyl, isopropyl, butyl, pentyl, hexyl and the like.

Suitable "tri(lower)alkylsilyl" may be trimethylsilyl, triethylsilyl and the like.

Suitable "trihalo(lower)alkanoyl" may be trichloroacetyl, trichloropropionyl and the like.

When free acid is used as an acylating agent, the acylation reaction may preferably be conducted in the presence of a conventional condensing agent such as N,N'-dicyclohexylcarbodiimide or the like.

This reaction is usually carried out in a solvent which does not adversely influence the reaction such as dioxane, chloroform, methylene chloride, tetrahydrofuran, pyridine or the like.

The reaction may also be carried out in the presence of an inorganic or organic base such as an alkali metal bicarbonate, tri(lower)alkylamine, pyridine, N-(lower)alkylmorpholine, N,N-di(lower)alkylbenzylamine, or the like.

The reaction temperature is not critical and the reaction can be carried out under cooling to heating.

In the present reaction, in case that the compound [II] having hydroxy for A is used as a starting compound, a compound of the formula : 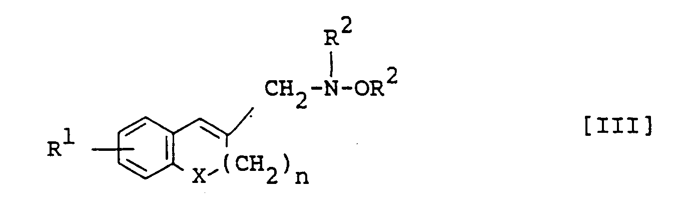
wherein R¹, R², X and n are each as defined above, may be obtained according to reaction conditions and in that case, the compound [III] is further subjected to an elimination reaction of acyl group to give the compound [Ia]. This case is also included within the scope of the present reaction.

The present elimination reaction can be carried out by a conventional manner such as hydrolysis or the like.

The hydrolysis is usually carried out in a conventional solvent such as an alcohol (e.g. methanol, ethanol, etc.), dioxane, tetrahydrofuran or any other organic solvent which does not adversely influence the reaction. These conventional solvents may also be used in a mixture with water.

This reaction may also be carried out in the presence of a base or an acid including Lewis acid. Suitable base may be an alkali metal [e.g. sodium, potassium, etc.], an alkaline earth metal [e.g. magnesium, calcium, etc.], the hydroxide or carbonate or bicarbonate thereof, alkali metal alkanoate [e.g. sodium acetate, etc.] or the like. Suitable acid may include an organic acid [e.g. formic acid, acetic acid, propionic acid, trichloroacetic acid, trifluoroacetic acid, etc.] and an inorganic acid [e.g. hydrochloric acid, hydrobromic acid, sulfuric acid, etc.] or the like.

The reaction temperature is not critical and the reaction can be carried out under cooling to heating.

### Process 2

The compound [Ia] can be prepared by subjecting the compound [Ib] to the deprotection reaction of hydroxy-protective group.

This deprotection reaction is preferably carried out in the presence of acid such as an organic acid (e.g. formic acid, acetic acid, propionic acid, trichloroacetic acid, etc.) and an inorganic acid (e.g. hydrochloric acid, hydrobromic acid, sulfuric acid, etc.).

The reaction is usually carried out in a conventional solvent such as water, an alcohol (e.g. methanol, ethanol, etc.), dioxane, tetrahydrofuran or any other organic solvent which does not adversely influence the reaction. These conventional solvents may also be used in a mixture with water.

The reaction temperature is not critical and the reaction can be carried out under cooling to heating.

Pharmaceutically acceptable salts of the compound [I] can be prepared by a conventional method, e.g., by treating the compound [I] with a base. Preferred examples of said base are the same as those exemplified in the explanation of pharmaceutically acceptable salts of the compound [I].

The starting compounds [II] are new and can be prepared by the processes as illustrated in the following reaction schemes. 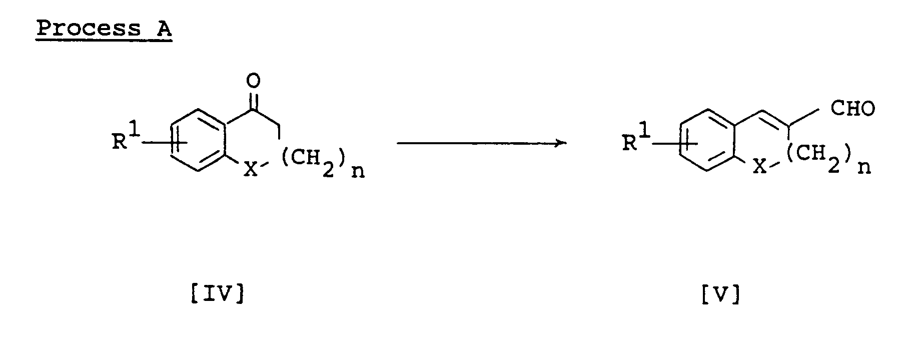 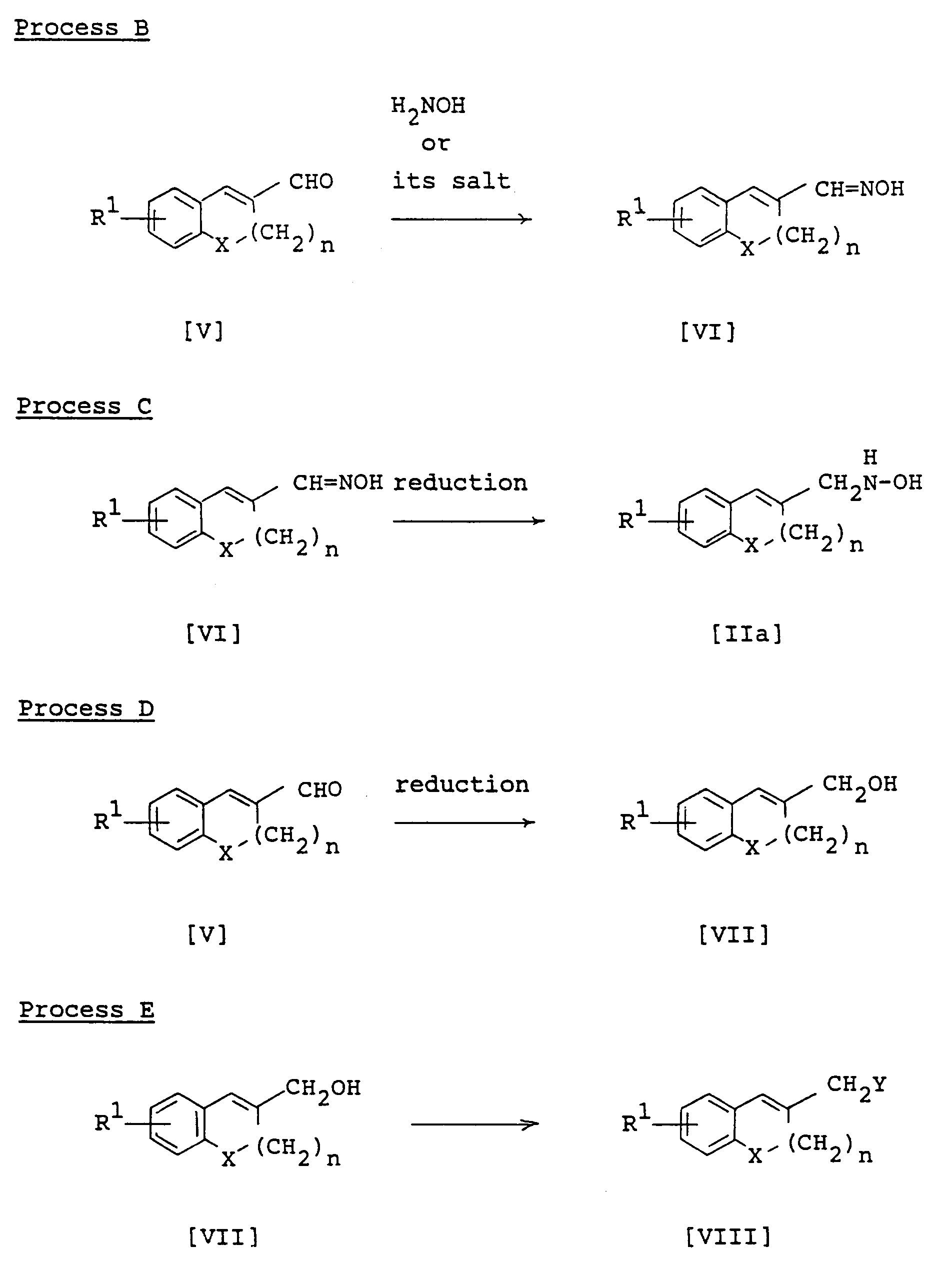 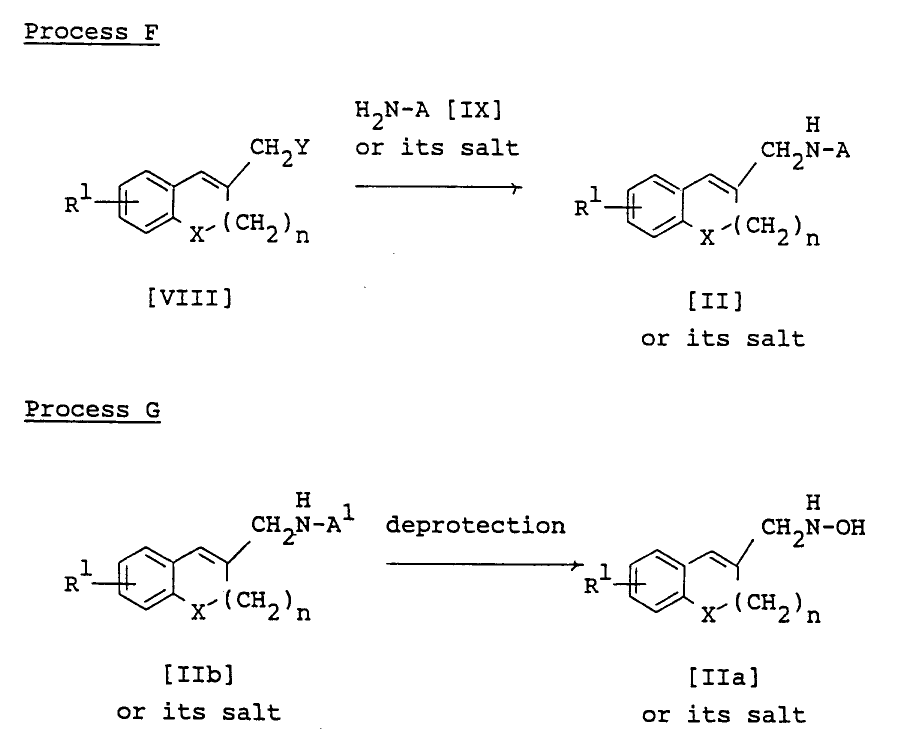
wherein
- R¹, X, n, A and A¹: are each as defined above, and
- Y: is acid residue.

Suitable "acid residue" may be halogen (e.g. chloro, fluoro, bromo and iodo), arenesulfonyloxy (e.g. benzenesulfonyloxy, tosyloxy, etc.), alkanesulfonyloxy (e.g. mesyloxy, ethanesulfonyloxy, etc.), and the like.

The above-mentioned processes for preparing the starting compounds [II] are explained in detail in the following.

### Process A

The compound [V] can be prepared by the following 3 steps :
1) the first step
   Reaction of a compound [IV] with a di(lower)alkoxy carbenium haloborate [e.g. dimethoxy carbenium fluoroborate, diethoxycarbenium fluoroborate, etc.] prepared by the reaction of tri(lower)alkyl orthoformate [e.g. trimethyl orthoformate, triethyl orthoformate, etc.] with boron trihalide [e.g. boron trifluoride, boron trichloride, etc.], which gives a compound of the formula : 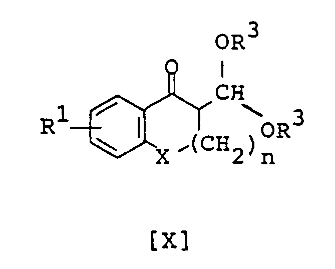 wherein
   - R¹, X and n: are each as defined above and
   - R³: is lower alkyl.
2) the 2nd step
   Reaction of the resulting compound [X] with a reducing agent such as aluminum hydride compound [e.g. lithium aluminum hydride, sodium aluminum hydride, etc.], borohydride compound [e.g. sodium borohydride, lithium borohydride, etc.] or the like, which gives a compound of the formula : 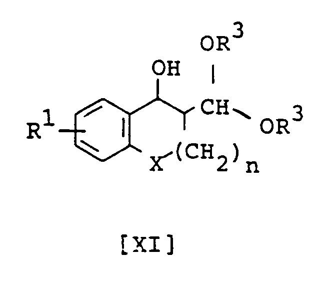 wherein
   - R¹, R³, X and n: are each as defined above.
3) the 3rd step
   Reaction of the resulting compound [XI] with an acid such as an organic acid [e.g. formic acid, acetic acid, propionic acid, trifluoroacetic acid, p-toluenesulfonic acid, etc.], an inorganic acid [e.g. hydrochloric acid, hydrobromic acid, etc.] or the like.

The reaction of the first step is carried out under cooling in a conventional solvent which does not adversely influence the reaction, such as dioxane, chloroform, methylene chloride, tetrahydrofuran, or the like.

The reaction may also be carried out in the presence of an organic base [e.g. diisopropylethylamine, etc.].

The reaction of the 2nd step is usually carried out at ambient temperature, or under warming or heating in a conventional solvent which does not adversely influence the reaction such as an alcohol (e.g. methanol; ethanol, etc.), dioxane, tetrahydrofuran, or the like.

The reaction temperature of the 3rd step is not critical and the reaction can be carried out under cooling to heating in a conventional solvent which does not adversely influence the reaction, such as water, an alcohol (e.g. methanol, ethanol, etc.) acetone, dioxane, acetonitrile, tetrahydrofuran, or the like. These conventional solvents may be used in a mixture with water.

### Process B

The compound [VI] can be prepared by reacting the compound [V] with hydroxylamine or its salt.

Suitable salts of hydroxylamine may include salt with an organic acid (e.g. formic acid, acetic acid, propionic acid, etc.) and an inorganic acid (e.g. hydrochloric acid, hydrobromic acid, sulfuric acid, etc.).

The reaction is usually carried out in a conventional solvent such as water, an alcohol (e.g. methanol, ethanol, etc.), acetone, dioxane, acetonitrile, chloroform, methylene chloride, ethylene chloride, tetrahydrofuran, ethyl acetate, N,N-dimethylformamide, pyridine or any other organic solvent which does not adversely influence the reaction. These conventional solvents may also be used in a mixture with water.

The reaction may also be carried out in the presence of an inorganic or organic base such as an alkali metal bicarbonate, tri(lower)alkylamine, pyridine, N-(lower)alkylmorpholine, N,N-di(lower)alkylbenzylamine, or the like.

The reaction temperature is not critical, and the reaction is usually carried out under cooling to warming.

### Process C

The compound [IIa] can be prepared by reacting the compound [VI] with a reducing agent.

The suitable reducing agent may be diborane, borane-organic amine complex (e.g. borane-pyridine complex etc.), alkali metal cyanoborohydride (e.g. sodium cyanoborohydride, lithium cyanoborohydride etc.) and the like.

The reaction is usually carried out in a conventional solvent such as an alcohol (e.g. methanol, ethanol, etc.), dioxane, tetrahydrofuran or any other organic solvent which does not adversely influence the reaction.

The reaction may also be carried out in an acidic condition and the reaction temperature is not critical, and the reaction is usually carried out under cooling to warming.

### Process D

The compound [VII] can be prepared by reacting the compound [V] with a reducing agent.

The suitable reducing agent may be a metal hydride compound such as aluminum hydride compound (e.g. lithium tri-t-butoxyaluminum hydride, etc.), borohydride compound (e.g. sodium borohydride, etc.), aluminum alkoxide (e.g. aluminum isoproxide, etc.) or the like.

The reaction is usually carried out in a conventional solvent, such as water, an alcohol (e.g. methanol, ethanol, propanol, isopropanol, etc.), tetrahydrofuran, or any other organic solvent which does not adversely influence the reaction, or a mixture thereof.

The reaction temperature is not critical, and the reaction can be carried out under cooling to heating.

### Process E

The compound [VIII] can be prepared by reacting the compound [VII] with an acid or its reactive derivative.

Suitable acid may include an organic acid such as arenesulfonic acid (e.g. benzenesulfonic acid, toluenesulfonic acid, etc.), alkanesulfonic acid (e.g. methanesulfonic acid, ethanesulfonic acid, etc.), haloalkanesulfonic acid (e.g. trifluoromethanesulfonic acid, etc.) and an inorganic acid (e.g. hydrochloric acid, hydrobromic acid, etc.) or the like.

Suitable reactive derivative of acid may be a conventional one such as arenesulfonyl halide (e.g. benzenesulfonyl chloride, toluenesulfonyl chloride, etc.), alkanesulfonyl halide (e.g. methanesulfonyl chloride, methanesulfonyl bromide, etc.) and an acid anhydride (e.g. trifluoromethanesulfonic acid anhydride, etc.) or the like.

The reaction is usually carried out in a conventional solvent, such as water, an alcohol (e.g. methanol, ethanol, propanol, isopropanol, etc.), chloroform, diethyl ether, n-hexane, tetrahydrofuran, dioxane, or any other organic solvent which does not adversely influence the reaction. These conventional solvents may also be used in a mixture with water.

The reaction temperature is not critical, and the reaction can be carried out under cooling to heating.

### Process F

The compound [II] or its salt can be prepared by reacting the compound [VIII] with the compound [IX] or its salt.

Suitable salt of the compound [II] and [IX] may be the same as those exemplified for hydroxylamine in Process B.

This reaction is usually carried out in a conventional solvent such as water, an alcohol (e.g. methanol, ethanol, etc.), acetone, dioxane, acetonitrile, chloroform, methylene chloride, ethylene chloride, tetrahydrofuran, N,N-dimethylformamide or any other organic solvent which does not adversely influence the reaction. These conventional solvents may also be used in a mixture with water.

The reaction may also be carried out in the presence of an inorganic base or organic base such as alkali metal carbonate (e.g. sodium carbonate, potassium carbonate, etc.); alkali metal bicarbonate (e.g. sodium bicarbonate, potassium bicarbonate, etc.), trialkylamine (e.g. triethylamine, etc.) or the like.

The reaction temperature is not critical and the reaction can be carried out under cooling to heating.

### Process G

The compound [IIa] or its salt can be prepared by subjecting the compound [IIb] or its salt to the deprotection reaction of hydroxy-protective group.

Suitable salt of the compounds [IIa] and [IIb] are the same as those exemplified for hydroxylamine in Process B.

This deprotection reaction is preferably carried out in the presence of an acid such as an organic acid (e.g. formic acid, acetic acid, propionic acid, trichloroacetic acid, etc.) and an inorganic acid (e.g. hydrochloric acid, hydrobromic acid, sulfuric acid, etc.).

The reaction is usually carried out in a conventional solvent such as water, an alcohol (e.g. methanol, ethanol, etc.), dioxane, tetrahydrofuran or any other organic solvent which does not adversely influence the reaction. These conventional solvents may also be used in a mixture with water.

The reaction temperature is not critical and the reaction can be carried out under cooling to heating.

The compounds obtained by the above processes can be isolated and purified by a conventional method such as pulverization, recrystallization, column chromatography, reprecipitation, or the like.

The object compounds [I] and pharmaceutically acceptable salts thereof possess strong inhibitory activities against 5-lipoxygenase (SRS-A synthesis) and are useful as an antiallergic agent or an antiinflammatory agent for human beings and animals, and more particularly are useful for treatment of asthma, psoriasis, hepatitis, pancreatitis, arthritis, nephritis, inflammatory bowel disease, septic shock, arteriosclerosis, myocardial infarction, cerebral vasospasm, rhinitis, conjunctivitis, dermatitis, rheumatism, peptic ulcer, gout or the like.

In order to illustrate the usefulness of the object compound [I], the pharmacological test data of some representative compounds of the compound [I] are shown in the following.

### Test compounds :

(a) N-[(3,4-Dihydro-5-phenoxy-2-naphthyl)methyl]-N-hydroxyacetamide
(b) N-[(5-Benzyloxy-3,4-dihydro-2-naphthyl)methyl]-N-hydroxyacetamide
(c) N-[[8-Benzyloxy-3-(2H-1-benzopyranyl)methyl]-N-hydroxyacetamide
(d) N-[[7-Benzyloxy-2-(1H-indenyl)]methyl]-N-hydroxyacetamide
(e) N-Hydroxy-N-methoxycarbonyl-(3,4-dihydro-5-phenoxy-2-naphthyl)methylamine
(f) N-[(3,4-Dihydro-5-phenoxy-2-naphthyl)methyl]-N-hydroxy-N'-ethylurea

### Test 1

### Test Method : Inhibitory activity of SRS-A (Slow Reacting Substance of Anaphylaxis) synthesis in rat polymorpholeukocyte (PMN) using the calcium ionophore

### Preparation of PMN from rat

Male Spraque-Dawley rats weighing 250-300 g were anesthetized with ether and each was injected intraperitoneally with 20 ml of 0.1% glycogen (from Oyster). After 20 hours the rats were sacrificed and PMN were recovered in rinse of the peritoneal cavity with 10 ml Dulbeccos PBS (components in G/L CaCℓ₂ 0.1, KH₂PO₄ 0.2, MgCℓ₂·6H₂O 0.1, NaCℓ 8.0, Na₂HPO₄·7H₂O 2.16; pH 7.4). These rinses were filtered through nylon wool filter and centrifuged for 5 min at 1000 xg.
The pellet was suspended in Dulbeccos PBS and centrifuged for 5 min at 1000 xg. The pellet was resuspended in Dulbeccos PBS and adjusted the cell concentration to 10⁷ cells/ml with Dulbeccos PBS.

### PMN stimulation

Samples were dissolved in ethanol and dispersed in Dulbeccos PBS to give a concentration of 10⁻¹⁰ to 10⁻⁵M. Antibiotic A23187; calcium ionophor (Dehring Diagnostics) (hereinafter referred to A23187) in DMSO (10mM) was, diluted with Dulbeccos PBS to give the concentration of 1mM. Aliquots of the cell suspension (1x10⁷ cells/ml, 0.98 ml) were equilibrated for 30 min at 37°C. Solution of sample (10 »ℓ) was added and incubated for 15 min at 37°C before the addition of 10 »ℓ of A 23187 solution. Thus the final incubation volume of 1 ml contained approximately 1x10⁷ cells, 10⁻¹⁰ to 10⁻⁵M samples and 10»M A23187. Incubation with A23187 were continued for 15 min at 37°C. The reactions were terminated by setting the assay tubes in ice bath to chill as rapidly as possible to 4°C. The test tubes were centrifuged at 1500 xg for 5 min at 4°C and decanted the supernatants into the tubes and kept cold prior to assaying.

### Determination of immunoreactive LTC4 (i-LTC4)

The concentration of i-LTC4 in the cell-free supernatants from the incubations were determined by specific radioimmunoassay. The mean values of i-LTC4 (these incubations were carried out in duplicate) of each sample were calculated and the effect of samples on the synthesis of the leukotrienes was presented as a percentage of the value in the absence of samples.

### Test Results

| Test Compound | IC₅₀ (M) |
|---|---|
| (a) | 4.1 x 10⁻⁸ |
| (b) | 7.4 x 10⁻⁸ |
| (c) | 8.5 x 10⁻⁸ |
| (d) | 4.0 x 10⁻⁸ |
| (e) | 3.2 x 10⁻⁸ |
| (f) | 2.4 x 10⁻⁸ |

### Test 2

### Method : Effect of compound on D-Galactosamine-induced hepatitis in rats

Acute hepatitis was induced in male Wistar rats at 6 weeks of age by the intraperitoneal injection of 80 mg/ml D-Galactosamine in saline at the volume of 5 ml/kg. Blood samples were taken in rats under ether anaesthesia 24 hours later.

After centrifugation for the separation of sera, the levels of GOT and GPT were determined spectrophotometrically according to Biochemical Analyzer TBA-20R (Toshiba).

Compound was dissolved in polyethylene glycol 400, and administered orally 3 hour before and after the injection of D-Galactosamine.

The levels of serum GOT and GPT in group of animals were measured as described above, and the mean value of each group calculated, including normal rats without D-Galactosamine injection.

The effect of compound was expressed as the percentage Inhibition at serum GOT and GPT, taking activity of control serum as 0% Inhibtion and that of normal serum as 100% Inhibition.

### Result

| Dose of compound (f) (mg/kg) | No. of animals | Inhibition (%) | |
|---|---|---|---|
| | | serum GOT | serum GPT |
| 1 | 8 | 38 | 42 |
| 10 | 9 | 40 | 37 |
| 32 | 10 | 67 | 64 |

Pharmaceutical compositions of this invention can be used in a conventional pharmaceutical forms such as powders, fine granules, granules, tablets, dragee, microcapsules, capsules, suppository, solution, suspension, emulsion, syrups and the like. It is administered by oral, parenteral or external route. If desired, diluents or disintegrators (e.g. sucrose, lactose, starch, crystalline cellulose, low-substituted hydroxypropyl cellulose, synthetic aluminum silicate, etc.), binding agents (e.g. cellulose, methylcellulose, hydroxypropylcellulose, hydroxypropylmethylcellulose, polypropylpyrrolidone, polyvinylpyrrolidone, gelatin, gum arabic, polyethyleneglycol, etc.), coloring agents, sweating agents, lubricant (e.g. magnesium stearate, etc.) or the like, may be dispensed with said composition.

The dosage of said composition of this invention depends on the patient's age, body weight, condition, etc., and it is generally administered e.g. by the oral route at the daily dose level of 100 mg to 10 g as the object compound [I] or its pharmaceutically acceptable salt, preferably 1 g to 5 g on the same basis, at the interval of 1 to 3 times a day. Typical unit doses may be 50 mg, 100 mg, 200 mg, 500 mg, 1 g and the like, although these are only examples and not limitative, of course.

The following Preparations and Examples are given for the purpose of illustrating this invention.

### Preparation 1

To a solution of triethyl orthoformate (0.37 ml) in methylene chloride (2 ml) was added dropwise borontrifluoride etherate (0.32 ml) at -30°C under a nitrogen atmosphere. The mixture was then allowed to warm to 0°C and stirred for 15 minutes. The resulting slurry of diethoxycarbenium fluoroborate was cooled to -78°C and 3,4-dihydro-5-phenoxy-1(2H)-naphthalenone (262 mg) was added in one portion followed by dropwise addition of N,N-diisopropylethylamine (0.57 ml) over a period of 15 minutes. After stirring at -78°C for 15 minutes, the mixture was stirred for one hour at -20 to -10°C and poured into saturated aqueous sodium bicarbonate solution. The separated aqueous layer was extracted with methylene chloride. The combined methylene chloride layers were washed with cooled 1N sulfuric acid, brine, saturated aqueous sodium bicarbonate solution, and brine. The methylene chloride layer was dried and concentrated in vacuo to give 2-diethoxymethyl-3,4-dihydro-5-phenoxy-1(2H)-naphthalenone as an oil (395 mg), which was dissolved in methanol (10 ml) and treated with sodium borohydride (94 mg) at 0°C for 10 minutes. The mixture was poured into ice water. The separated oil was extracted with ethyl acetate. The organic layer was washed with brine, dried, and evaporated. The resulting residue containing 2-diethoxymethyl-1,2,3,4-tetrahydro-5-phenoxy-1-naphthol was dissolved in a mixture of dioxane (10 ml) and aqueous 2N-hydrochloric acid (5 ml) and stirred at 70°C for one and half hours. The mixture was poured into ice water. The separated oil was extracted with diethyl ether. The extract was washed with brine, dried, and concentrated to give an oil, which was purified by column chromatography on silica gel (elution by n-hexane/ethyl acetate = 5/1) to yield 3,4-dihydro-5-phenoxy-2-naphthalenecarbaldehyde (214 mg) as an oil.
- IR (neat) :: 2800, 1660, 1620, 1580 cm⁻¹
- NMR (CDCℓ₃, δ) :: 2.52 (2H, t, J=8H), 2.88 (2H, t, J=8Hz), 6.90-7.38 (9H, m), 9.71 (1H, s)
The following compounds (Preparations 2-1) to 2-13)) were obtained according to a similar manner to that of Preparation 1.

### Preparation 2

1) 3,4-Dihydro-7-phenoxy-2-naphthalenecarbaldehyde
   - mp :: 91-93°C
   - IR (Nujol) :: 1660, 1580 cm⁻¹
   - NMR (CDCℓ₃, δ) :: 2.57 (2H, t, J=8Hz), 2.88 (2H, t, J=8Hz), 6.98 (1H, s), 7.01 (2H, d, J=8Hz), 7.08-7.20 (4H, m), 7.35 (1H, d, J=8Hz), 7.39 (1H, t, J=8Hz), 9.63 (1H, s)
2) 6-Benzyloxy-3,4-dihydro-2-naphthalenecarbaldehyde
   - mp :: 68-70°C
   - IR (Nujol) :: 1670, 1620, 1600 cm⁻¹
   - NMR (CDCℓ₃, δ) :: 2.55 (2H, t, J=8Hz), 2.88 (2H, t, J=8Hz), 5.99 (2H, s), 6.80-6.88 (2H, m), 7.21-7.25 (2H, m), 7.38-7.48 (5H, m), 9.62 (1H, s)
3) 7-Benzyloxy-3,4-dihydro-2-naphthalenecarbaldehyde
   oil
   - IR (Neat) :: 1660, 1620, 1600 cm⁻¹
   - NMR (CDCℓ₃, δ) :: 2.53 (2H, t, J=8Hz), 2.82 (2H, t, J=8Hz), 5.08 (2H, s), 6.90-7.45 (9H, m), 9.67 (1H, s)
4) 5-Benzyloxy-3,4-dihydro-2-naphthalenecarbaldehyde
   - mp :: 116-118°C
   - IR (CHCℓ₃) :: 1665, 1630, 1570 cm⁻¹
   - NMR (CDCℓ₃, δ) :: 2.55 (2H, t, J=8Hz), 2.92 (2H, t, J=8Hz), 5.11 (2H, s), 6.90-7.05 (2H, m), 7.15-7.55 (7H, m), 9.68 (1H, s)
5) 3,4-Dihydro-7-phenyl-2-naphthalenecarbaldehyde
   - mp :: 105-107°C
   - IR (Nujol) :: 1660, 1620, 1270 cm⁻¹
   - NMR (CDCℓ₃, δ) :: 2.62 (2H, t, J=8Hz), 2.92 (2H, t, J=8Hz), 7.27-7.62 (9H, m), 9.68 (1H, s)
6) 7-Benzyl-3,4-dihydro-2-naphthalenecarbaldehyde
   oil
   - IR (Neat) :: 2930, 1660, 1620 cm⁻¹
   - NMR (CDCℓ₃, δ) :: 2.52 (2H, t, J=8Hz), 2.85 (2H, t, J=8Hz), 4.00 (2H, s), 7.02-7.34 (9H, m), 9.62 (1H, s)
7) 8-Benzyloxy-2H-1-benzopyran-3-carbaldehyde
   - mp :: 97-98°C
   - IR (Nujol) :: 1660, 1640, 1600 cm⁻¹
   - NMR (CDCℓ₃, δ) :: 5.08 (2H, s), 5.18 (2H, s), 6.83 (1H, d, J=5Hz), 6.88 (1H, s), 6.92-6.98 (1H, m), 7.23-7.46 (6H, m), 9.61 (1H, s)
8) 8-Benzyloxy-2H-1-benzothiopyran-3-carbaldehyde
   - mp :: 118-120°C
   - IR (CHCℓ₃) :: 3000, 2820, 1675, 1630 cm⁻¹
   - NMR (CDCℓ₃, δ) :: 3.72 (2H, s), 5.20 (2H, s), 6.91 (1H, d, J=7Hz), 6.99 (1H, d, J=7Hz), 7.10 (1H, t, J=7Hz), 7.28 (1H, s), 7.30-7.60 (5H, m), 9.65 (1H, s)
9) 7-Benzyloxy-1H-indene-2-carbaldehyde
   - mp :: 99-101°C
   - IR (Nujol) :: 1650, 1580 cm⁻¹
   - NMR (CDCℓ₃, δ) :: 3.71 (2H, s), 5.21 (2H, s), 6.98 (1H, d, J=8Hz), 7.22-7.50 (7H, m), 7.72 (1H, s), 10.01 (1H, s)
10) 2-Benzyloxy-6,7-dihydro-5H-benzocyclohepten-8-carbaldehyde
   - mp :: 71-72°C
   - IR (CHCℓ₃) :: 2950, 1675, 1630, 1605 cm⁻¹
   - NMR (CDCℓ₃, δ) :: 1.90-2.10 (2H, m), 2.59 (2H, t, J=8Hz), 2.85 (2H, t, J=8Hz), 5.09 (2H, s), 6.90 (1H, d, J=7Hz), 7.00 (1H, s), 7.10 (1H, d, J=7Hz), 7.17 (1H, s), 7.25-7.50 (5H, m), 9.58 (1H, s)
11) 8-Phenoxy-2H-1-benzopyran-3-carbaldehyde
   - mp :: 123-124°C
   - IR (Nujol) :: 1660, 1630, 1595, 1490 cm⁻¹
   - NMR (CDCl₃, δ) :: 5.05 (2H, s), 6.88-7.36 (9H, m), 9.61 (1H, s)
12) 3,4-Dihydro-7-phenylthio-2-naphthalenecarbaldehyde
   - mp :: 102-105°C
   - IR (CHCl₃) :: 3000, 2820, 1667, 1622, 1475, 1382, 1300, 1170, 1150, 1115 cm⁻¹
   - NMR (CDCl₃, δ) :: 2.57 (2H, t, J=7Hz), 2.85 (2H, t, J=7Hz), 7.10-7.45 (9H, m), 9.64 (1H, s)
13) 7-Phenoxy-1H-indene-2-carbaldehyde
   - mp :: 85-86°C
   - IR (Nujol) :: 1660, 1580, 1560, 1490, 1390, 1240, 1210, 1130 cm⁻¹
   - NMR (CDCl₃, δ) :: 3.59 (2H, s), 6.97-7.14 (4H, m), 7.25-7.43 (4H, m), 7.78 (1H, s), 9.97 (1H, s)

### Preparation 3

A mixture of 3,4-dihydro-5-phenoxy-2-naphthalenecarbaldehyde (194 mg), hydroxylamine hydrochloride (162 mg), and sodium bicarbonate (196 mg) in N,N-dimethylformamide (10 ml) was stirred at 70°C for half an hour. The mixture was cooled to atmospheric temperature and poured into water. The separated oil was extracted with diethyl ether (x2). The combined extracts were washed with brine, dried, and concentrated in vacuo. The residue was crystallized from ethanol to yield 3,4-dihydro-5-phenoxy-2-naphthalenecarbaldehyde oxime (211 mg).
- mp :: 159-161°C
- IR (Nujol) :: 3250, 1580, 1560, 1280 cm⁻¹
- NMR (CDCℓ₃, δ) :: 2.55 (2H, t, J=8Hz), 2.85 (2H, t, J=8Hz), 6.63 (1H, s), 6.82-7.20 (6H, m), 7.35 (2H, m), 7.92 (1H, s), 8.07 (1H, s)
The following compounds (Preparations 4-1) to 4-12)) were obtained according to a similar manner to that of Preparation 3.

### Preparation 4

1) 3,4-Dihydro-7-phenoxy-2-naphthalenecarbaldehyde oxime
   - mp :: 133-135°C
   - IR (Nujol) :: 3250, 1590, 1560 cm⁻¹
   - NMR (CDCℓ₃, δ) :: 2.62 (2H, t, J=8Hz), 2.85 (2H, t, J=8Hz), 6.57 (1H, s), 6.77 (1H, s), 6.85 (1H, d, J=8Hz), 7.00 (2H, d, J=8Hz), 7.12 (2H, d, J=8Hz), 7.30-7.40 (2H, m), 7.90 (1H, s)
2) 6-Benzyloxy-3,4-dihydro-2-naphthalenecarbaldehyde oxime
   - mp :: 135-137°C
   - IR (Nujol) :: 3250, 3200, 1600, 1560 cm⁻¹
   - NMR (CDCℓ₃, δ) :: 2.59 (2H, t, J=8Hz), 2.84 (2H, t, J=8Hz), 5.07 (2H, s), 6.62 (1H, s), 6.78 (1H, d, J=10Hz), 6.82 (1H, s), 7.06 (1H, d, J=10Hz), 7.32-7.45 (5H, m), 7.88 (1H, s)
3) 7-Benzyloxy-3,4-dihydro-2-naphthalenecarbaldehyde oxime
   - mp :: 133-135°C
   - IR (Nujol) :: 3325, 1600, 1560 cm⁻¹
   - NMR (CDCℓ₃, δ) :: 2.59 (2H, t, J=8Hz), 2.81 (2H, t, J=8Hz), 5.07 (2H, s), 6.60 (1H, s), 6.79 (1H, s), 6.80 (1H, d, J=10Hz), 7.07 (1H, d, J=10Hz), 7.32-7.47 (5H, m), 7.92 (1H, s)
4) 5-Benzyloxy-3,4-dihydro-2-naphthalenecarbaldehyde oxime
   - mp :: 139-141°C
   - IR (CHCℓ₃) :: 3600, 3300, 1620, 1605 cm⁻¹
   - NMR (CDCℓ₃, δ) :: 2.59 (2H, t, J=8Hz), 2.94 (2H, t, J=8Hz), 5.10 (2H, s), 6.63 (1H, s), 6.78 (1H, d, J=7Hz), 6.87 (1H, d, J=7Hz), 7.12 (1H, t, J=7Hz), 7.25-7.55 (5H, m), 7.92 (1H, s)
5) 3,4-Dihydro-7-phenyl-2-naphthalenecarbaldehyde oxime
   - mp :: 172-174°C
   - IR (Nujol) :: 3250, 1600, 1300 cm⁻¹
   - NMR (DMSO-d₆, δ) :: 2.57 (2H, t, J=8Hz), 2.82 (2H, t, J=8Hz), 6.83 (1H, s), 7.22-7.50 (6H, m), 7.63 (2H, d, J=8Hz), 7.97 (1H, s), 11.18 (1H, s)
6) 7-Benzyl-3,4-dihydro-2-naphthalenecarbaldehyde oxime
   - mp :: 138-140°C
   - IR (Nujol) :: 3200, 1600, 1490 cm⁻¹
   - NMR (CDCℓ₃, δ) :: 2.58 (2H, t, J=8Hz), 2.84 (2H, t, J=8Hz), 3.95 (2H, s), 6.59 (1H, s), 6.97 (1H, s), 6.99-7.32 (7H, m), 7.90 (1H, s)
7) 8-Benzyloxy-2H-1-benzopyran-3-carbaldehyde oxime
   - mp :: 121-123°C
   - IR (Nujol) :: 3250, 1630, 1570 cm⁻¹
   - NMR (CDCℓ₃, δ) :: 5.08 (2H, s), 5.18 (2H, s), 6.62 (1H, s), 6.68-6.78 (3H, m), 7.30-7.48 (5H, m), 7.82 (1H, s)
8) 8-Benzyloxy-2H-1-benzothiopyran-3-carbaldehyde oxime
   - mp :: 135-143°C
   - IR (CHCℓ₃) :: 3600, 3300, 1625, 1600 cm⁻¹
   - NMR (CDCℓ₃, δ) :: 3.76 (2H, s), 5.15 (2H, s), 6.63 (1H, s), 6.80 (1H, d, J=7Hz), 6.83 (1H, d, J=7Hz), 7.00 (1H, t, J=7Hz), 7.20-7.70 (5H, m), 7.91 (1H, s)
9) 7-Benzyloxy-1H-indene-2-carbaldehyde oxime
   oil
   - IR (Neat) :: 3300, 1590, 1260 cm⁻¹
   - NMR (CDCℓ₃, δ) :: 3.67 (2H, s), 5.19 (2H, s), 6.82 (1H, d, J=8Hz), 7.02-7.50 (8H, m), 8.15 (1H, s)
10) 2-Benzyloxy-6,7-dihydro-5H-benzocyclohepten-8-carbaldehyde oxime
   - mp :: 123-126°C
   - IR (CHCℓ₃) :: 3600, 3300, 2940, 1605 cm⁻¹
   - NMR (DMSO-d₆, δ) :: 1.80-2.00 (2H, m), 2.53 (2H, t, J=7Hz), 2.72 (2H, t, J=7Hz), 5.09 (2H, s), 6.68 (1H, s), 6.81 (1H, d, J=8Hz), 6.92 (1H, s), 7.08 (1H, d, J=8Hz), 7.25-7.50 (5H, m), 7.86 (1H, s)
11) 8-Phenoxy-2H-1-benzopyran-3-carbaldehyde oxime
   - mp :: 123-126°C
   - IR (Nujol) :: 3250, 1620, 1585, 1570, 1490, 1210 cm⁻¹
   - NMR (CDCl₃, δ) :: 5.50 (2H, s), 6.65 (1H, s), 6.87-7.36 (8H, m), 7.83 (1H, s)
12) 3,4-Dihydro-7-phenylthio-2-naphthalenecarbaldehyde oxime
   - mp :: 166-173°C
   - IR (CHCl₃) :: 3580, 3200, 1670, 1475, 950 cm⁻¹
   - NMR (DMSO-d₆, δ) :: 2.54 (2H, t, J=7Hz), 2.80 (2H, t, J=7Hz), 6.70 (1H, s), 7.20-7.45 (8H, m), 7.88 (1H, s)

### Preparation 5

To a solution of 3,4-dihydro-5-phenoxy-2-naphthalenecarbaldehyde oxime (194 mg) in ethanol (25 ml) was added sodium cyanoborohydride (322 mg) in several portions at ambient temperature, adjusting the pH to 3 by the addition of methanolic hydrogen chloride solution. After stirring for 2 hours at ambient temperature, the mixture was quenched with saturated aqueous ammonium chloride solution and the pH of the mixture was adjusted to 10 with aqueous 1N-sodium hydroxide solution. The mixture was extracted with ethyl acetate. The extract was washed with brine, dried, and evaporated to give a solid. The solid was crystallized from a mixture of ether and isopropyl ether to yield N-hydroxy-(3,4-dihydro-5-phenoxy-2-naphthyl)methylamine (95 mg).
- mp:: 92-95°C
- IR (Nujol) :: 3230, 1590, 1560, 1240, 1020 cm⁻¹
- NMR (CDCℓ₃, δ) :: 2.28 (2H, t, J=8Hz), 2.80 (2H, t, J=8Hz), 3.65 (2H, s), 6.52 (1H, s), 6.77-6.92 (4H, m), 7.00-7.18 (2H, m), 7.30-7.36 (2H, m)
The following compounds (Preparations 6-1) to 6-12)) were obtained according to a similar manner to that of Preparation 5.

### Preparation 6

1) N-Hydroxy-(3,4-dihydro-7-phenoxy-2-naphthyl)methylamine
   oil
   - IR (Neat) :: 3250, 2930, 1720, 1590 cm⁻¹
   - NMR (CDCℓ₃, δ) :: 2.36 (2H, t, J=8Hz), 2.80 (2H, t, J=8Hz), 3.65 (2H, s), 4.70 (2H, br s), 6.35 (1H, s), 6.70 (1H, s), 6.78 (1H, d, J=8Hz), 6.98-7.10 (4H, m), 7.28 (1H, s), 7.31 (1H, t, J=8Hz)
2) N-Hydroxy-(6-benzyloxy-3,4-dihydro-2-naphthyl)methylamine
   - mp :: 88-90°C
   - IR (Nujol) :: 3250, 1600, 1560 cm⁻¹
   - NMR (CDCℓ₃, δ) :: 2.30 (2H, t, J=8Hz), 2.82 (2H, t, J=8Hz), 3.67 (2H, s), 5.09 (2H, s), 6.37 (1H, s), 6.72 (1H, d, J=10Hz), 6.80 (1H, s), 6.93 (1H, d, J=10Hz), 7.32-7.50 (5H, m)
3) N-Hydroxy-(7-benzyloxy-3,4-dihydro-2-naphthyl)methylamine
   - mp :: 76-78°C
   - IR (Nujol) :: 3250, 1600 cm⁻¹
   - NMR (CDCℓ₃, δ) :: 2.31 (2H, t, J=8Hz), 2.80 (2H, t, J=8Hz), 3.68 (2H, s), 5.05 (2H, s), 6.38 (1H, s), 6.70 (1H, s), 6.72 (1H, d, J=10Hz), 7.00 (1H, d, J=10Hz), 7.30-7.43 (5H, m)
4) N-Hydroxy-(5-benzyloxy-3,4-dihydro-2-naphthyl)methylamine
   - mp :: 91-92.5°C
   - IR (CHCℓ₃) :: 3600, 3300, 1600, 1575 cm⁻¹
   - NMR (CDCℓ₃, δ) :: 2.32 (2H, t, J=8Hz), 2.91 (2H, t, J=8Hz), 3.68 (2H, s), 4.57 (2H, br.s), 5.09 (2H, s), 6.41 (1H, s), 6.70 (1H, d, J=7Hz), 6.80 (1H, d, J=7Hz), 7.09 (1H, t, J=7Hz), 7.25-7.60 (5H, m)
5) N-Hydroxy-(3,4-dihydro-7-phenyl-2-naphthyl)methylamine
   - mp :: 134-136°C
   - IR (Nujol) :: 3250, 1600, 1310 cm⁻¹
   - NMR (CD₃OD, δ) :: 2.41 (2H, t, J=8Hz), 2.90 (2H, t, J=8Hz), 3.71 (2H, s), 6.56 (1H, s), 7.17-7.48 (6H, m), 7.59 (2H, d, J=8Hz)
6) N-Hydroxy-(7-benzyl-3,4-dihydro-2-naphthyl)methylamine
   oil
   - IR (CHCℓ₃) :: 3300, 1600, 1490 cm⁻¹
   - NMR (CDCℓ₃,δ) :: 2.28 (2H, t, J=8Hz), 2.81 (2H, t, J=8Hz), 3.60 (2H, s), 3.91 (2H, s), 6.33 (1H, s), 6.86 (1H, s), 6.93-7.32 (7H, m)
7) N-Hydroxy-[8-benzyloxy-3-(2H-1-benzopyranyl)]methylamine
   oil
   - IR (Neat) :: 3250, 1570 cm⁻¹
   - NMR (CDCℓ₃, δ) :: 3.69 (2H, s), 4.87 (2H, s), 5.07 (2H, s), 6.35-6.82 (3H, m), 7.30-7.43 (7H, m)
8) N-Hydroxy-[8-benzyloxy-3-(2H-1-benzothiopyranyl)]methylamine
   - mp :: 108.5-110°C
   - IR (CHCℓ₃) :: 3600, 3300, 3000, 2880, 1560 cm⁻¹
   - NMR (CDCℓ₃, δ) :: 3.47 (2H, s), 3.69 (2H, s), 5.12 (2H, s), 5.43 (2H, br.s), 6.43 (1H, s), 6.55-6.80 (2H, m), 6.94 (1H, t, J=7Hz), 7.10-7.60 (5H, m)
9) N-Hydroxy-[7-benzyloxy-2-(1H-indenyl)]methylamine
   oil
   - IR (Neat) :: 3250, 2900, 1600, 1580 cm⁻¹
   - NMR (CDCℓ₃, δ) :: 3.45 (2H, s), 3.98 (2H, s), 5.12 (2H, s), 6.73-6.83 (2H, m), 6.95-7.48 (7H, m)
10) N-Hydroxy-[2-benzyloxy-6,7-dihydro-8-(5H-benzocycloheptenyl)]methylamine
   - mp :: 76-77°C
   - IR (CHCℓ₃) :: 3600, 3300, 2950, 1605 cm⁻¹
   - NMR (CDCℓ₃, δ) :: 1.90-2.10 (2H, m), 2.35 (2H, t, J=8Hz), 2.71 (2H, t, J=7Hz), 3.62 (2H, s), 4.73 (2H, br.s), 5.04 (2H, s), 6.42 (1H, s), 6.71 (1H, d, J=7Hz), 6.79 (1H, s), 7.00 (1H, d, J=7Hz), 7.25-7.50 (5H, m)
11) N-Hydroxy-[8-phenoxy-3-(2H-1-benzopyranyl)]methylamine
   oil
   - IR (CHCl₃) :: 3580, 3270, 3000, 2850, 1590, 1575, 1490, 1472, 1270, 1245, 1200, 1040 cm⁻¹
   - NMR (CDCl₃, δ) :: 3.60 (2H, s), 4.80 (2H, s), 5.01 (2H, br.s), 6.42 (1H, s), 6.75-6.90 (3H, m), 6.90-7.15 (3H, m), 7.20-7.40 (2H, m)
12) N-Hydroxy-(3,4-dihydro-7-phenylthio-2-naphthyl)methylamine
   - mp :: 74-75°C
   - IR (CHCl₃) :: 3600, 3270, 3000, 2930, 1580, 1490, 1305 cm⁻¹
   - NMR (CDCl₃, δ) :: 2.33 (2H, t, J=7Hz), 2.81 (2H, t, J=7Hz), 3.64 (2H, s), 5.20 (2H, br.s), 6.35 (1H, s), 6.90-7.40 (8H, m)

### Preparation 7

To a solution of 3,4-dihydro-5-phenoxy-2-naphthalenecarbaldehyde (4.11 g) in a mixture of methanol (40 ml) and tetrahydrofuran (30 ml) was added sodium borohydride (0.625 g) at 0°C under a nitrogen atmosphere. After stirring at 0°C for 10 minutes, the mixture was poured into ice water. The separated oil was extracted with diethyl ether. The extract was washed with brine, dried, and concentrated in vacuo. The residue was crystallized from a mixture of diisopropyl ether and n-hexane to yield 3,4-dihydro-5-phenoxy-2-naphthylmethanol (3.24 g).
- mp :: 84-86°C
- IR (Nujol) :: 3250, 1590, 1570, 1490, 1340, 1250, 1220, 1040, 1020 cm⁻¹
- NMR (CDCl₃, δ) :: 2.27 (2H, t, J=8Hz), 2.83 (2H, t, J=8Hz), 4.27 (2H, s), 6.50 (1H, s), 6.80-6.93 (4H, m), 7.00-7.18 (12H, m), 7.27-7.36 (2H, m)
The following compound (Preparation 8) was obtained according to a similar manner to that of Preparation 7.

### Preparation 8

### 7-Phenoxy-2-(1H-indenyl)methanol oil

- IR (CHCl₃) :: 3600, 3420, 3050, 3000, 2860, 1595, 1575, 1490, 1465, 1280, 1240, 1160, 1020 cm⁻¹
- NMR (CDCl₃, δ) :: 3.26 (2H, s), 4.51 (2H, s), 6.70-6.85 (2H, m), 6.90-7.40 (7H, m)

### Preparation 9

To a solution of 3,4-dihydro-5-phenoxy-2-naphthylmethanol (3.24 g) in a mixture of diethyl ether (60 ml) and n-hexane (60 ml) was added 48% hydrobromic acid (60 ml) at 0°C and the mixture was stirred at 0°C for 5 minutes and at ambient temperature for 1 hour. To the mixture was added 48% hydrobromic acid (30 ml). The mixture was stirred at ambient temperature for 15 minutes and poured into ice water. The organic layer was separated and the aqueous layer was extracted with diethyl ether. The combined organic extracts were washed with brine, dried, and concentrated in vacuo to give 2-bromomethyl-3,4-dihydro-5-phenoxynaphthalene (3.93 g).
- mp :: 59-61°C
- IR (Nujol) :: 1600, 1560, 1490, 1420, 1330, 1240, 1205, 1030 cm⁻¹
- NMR (CDCl₃, δ) :: 2.40 (2H, t, J=8Hz), 2.82 (2H, t, J=8Hz), 4.13 (2H, s), 6.60 (1H, s), 6.81-6.91 (4H, m), 6.99-7.17 (2H, m), 7.70-7.84 (2H, m)
The following compound (Preparation 10) was obtained according to a similar manner to that of Preparation 9.

### Preparation 10

### 2-Bromomethyl-7-phenoxy-1H-indene oil

- IR (CHCl₃) :: 3050, 3000, 1590, 1570, 1490, 1470, 1280, 1240, 1025 cm⁻¹
- NMR (CDCl₃, δ) :: 3.42 (2H, s), 4.38 (2H, s), 6.79 (1H, d, J=8Hz), 6.86 (1H, s), 6.90-7.40 (7H, m)

### Preparation 11

To the mixture of 2-bromomethyl-3,4-dihydro-5-phenoxynaphthalene (3.87 g) and potassium carbonate (2.545 g) in N,N-dimethylformamide (40 ml) was added O-(tetrahydropyran-2-yl)hydroxylamine (4.314 g). The mixture was stirred at ambient temperature overnight and poured into ice water. The separated oil was extracted with diethyl ether and the extract was washed with brine, dried, and concentrated in vacuo. The residue was purified by column chromatography on silica gel (elution by n-hexane/ethyl acetate = 3/1) to yield N-(3,4-dihydro-5-phenoxy-2-naphthyl)methyl-O-(tetrahydropyran-2-yl)hydroxylamine (4.08 g) as an oil.
- IR (Neat) :: 3250, 2950, 2850, 1595, 1575, 1490, 1460, 1240, 1220, 1030, 745 cm⁻¹
- NMR (CDCl₃, δ) :: 1.52-1.87 (6H, m), 2.32 (2H, t, J=8Hz), 2.78 (2H, t, J=8Hz), 3.51-3.62 (1H, m), 3.69 (2H, s), 3.90-3.99 (1H, m), 4.82-4.86 (1H, m), 6.47 (1H, s), 6.77-6.93 (4H, m), 7.00-7.15 (2H, m), 7.28-7.33 (2H, m)
The following compound (Preparation 12) was obtained according to a similar manner to that of Preparation 11.

### Preparation 12

### N-[7-Phenoxy-2-(1H-indenyl)]methyl-O-(tetrahydropyran-2-yl)hydroxylamine oil

- IR (CHCl₃) :: 2950, 2850, 1590, 1570, 1490, 1465, 1272, 1235, 1105, 1070, 1020, 900 cm⁻¹
- NMR (CDCl₃, δ) :: 1.35-1.80 (6H, m), 3.29 (2H, s), 3.40-3.60 (1H, m), 3.75-3.90 (1H, m), 3.94 (2H, s), 4.78 (1H, m), 6.71 (1H, s), 6.75 (1H, d, J=8Hz), 6.85-7.40 (7H, m)

### Preparation 13

To the solution of N-[7-phenoxy-2-(1H-indenyl)]methyl-O-(tetrahydropyran-2-yl)hydroxylamine (549 mg) in methanol (9 ml) was added concentrated hydrochloric acid (0.82 ml) at 0°C. The solution was stirred at ambient temperature for 3 hours and then concentrated hydrochloric acid (0.4 ml) was added to the solution. The solution was stirred at ambient temperature for 3 hours and concentrated hydrochloric acid (0.2 ml) was added to the solution. The solution was stirred for 30 minutes and poured into ice-water. Then, aqueous saturated sodium bicarbonate was added until the mixture was slightly basic. The mixture was extracted with ethyl acetate and the extract was washed with brine, dried and concentrated in vacuo. The residue was crystallized from a mixture of diethyl ether and n-hexane to yield N-hydroxy-[7-phenoxy-2-(1H-indenyl)]methylamine (228 mg).
- mp :: 99-100°C
- IR (CHCl₃) :: 3600, 3270, 1595, 1575, 1490, 1470, 1280, 1240 cm⁻¹
- NMR (CDCl₃, δ) :: 3.39 (2H, s), 3.89 (2H, s), 4.02 (2H, br.s), 6.65-6.85 (2H, m), 6.90-7.40 (7H, m)

### Example 1

To a mixture of N-hydroxy-(3,4-dihydro-5-phenoxy-2-naphthyl)methylamine (85 mg) and pyridine (25 mg) in methylene chloride (10 ml) was added acetic anhydride (98 mg) in one portion at ambient temperature. The mixture was stirred for half an hour at ambient temperature and quenched with ice water. The organic layer was washed with aqueous 1N-hydrochloric acid, brine, aqueous sodium bicarbonate solution, and brine successively. The solution was dried and concentrated in vacuo to give N-[(3,4-dihydro-5-phenoxy-2-naphthyl)methyl]-N-acetoxyacetamide as an oil (125 mg), which was dissolved in methanol and treated with aqueous 1N-sodium hydroxide solution for 15 minutes at ambient temperature. The mixture was acidified with concentrated hydrochloric acid. The separated oil was extracted with ethyl acetate. The organic layer was washed with brine, dried, and evaporated to give a solid. The solid was crystallized from a mixture of ethyl acetate and n-hexane to yield N-[(3,4-dihydro-5-phenoxy-2-naphthyl)methyl]-N-hydroxyacetamide (71 mg).
- mp :: 148-149°C
- IR (Nujol) :: 3150, 1590, 1570, 1340 cm⁻¹
- NMR (CDCl₃, δ) :: 2.15 (3H, s), 2.31 (2H, t, J=8Hz), 2.88 (2H, t, J=8Hz), 4.40 (2H, s), 6.43 (1H, s), 6.80-6.90 (4H, m), 7.00-7.18 (2H, m), 7.34 (2H, t, J=8Hz)
The following compounds (Examples 2-1) to 2-13)) were obtained according to a similar manner to that of Example 1.

### Example 2

1) N-[(3,4-Dihydro-7-phenoxy-2-naphthyl)methyl]-N-hydroxyacetamide
   oil
   - IR (Neat) :: 3150, 2930, 1600 cm⁻¹
   - NMR (CDCℓ₃, δ) :: 2.14 (3H, s), 2.28 (2H, t, J=8Hz), 2.83 (2H, t, J=8Hz), 4.37 (2H, s), 6.29 (1H, s), 6.70 (1H, s), 6.80 (1H, d, J=8Hz), 6.98-7.12 (4H, m), 7.33 (2H, t, J=8Hz)
2) N-[(6-Benzyloxy-3,4-dihydro-2-naphthyl)methyl]-N-hydroxyacetamide
   - mp :: 102-104°C
   - IR (Nujol) :: 3150, 1610, 1300, 1270 cm⁻¹
   - NMR (CDCℓ₃, δ) :: 2.15 (3H, s), 2.29 (2H, t, J=8Hz), 2.82 (2H, t, J=8Hz), 4.32 (2H, s), 5.05 (2H, s), 6.33 (1H, s), 6.73 (1H, d, J=10Hz), 6.80 (1H, s), 6.95 (1H, d, J=10Hz), 7.32-7.47 (5H, m), 8.42 (1H, br.s)
3) N-[(7-Benzyloxy-3,4-dihydro-2-naphthyl)methyl]-N-hydroxyacetamide
   - mp :: 139-140°C
   - IR (Nujol) :: 3150, 1620, 1600 cm⁻¹
   - NMR (CDCℓ₃, δ) :: 2.15 (3H, s), 2.28 (2H, t, J=8Hz), 2.80 (2H, t, J=8Hz), 4.35 (2H, s), 5.07 (2H, s), 6.32 (1H, s), 6.71 (1H, s), 6.76 (1H, d, J=10Hz), 7.02 (1H, d, J=10Hz), 7.31-7.48 (5H, m), 8.45 (1H, br.s)
4) N-[(5-Benzyloxy-3,4-dihydro-2-naphthyl)methyl]-N-hydroxyacetamide
   - mp :: 105-106.5°C
   - IR (CHCℓ₃) :: 3270, 1625, 1575 cm⁻¹
   - NMR (CD₃OD, δ) :: 2.17 (3H, s), 2.22 (2H, t, J=8Hz), 2.87 (2H, t, J=8Hz), 4.33 (2H, s), 5.08 (2H, s), 6.37 (1H, s), 6.68 (1H, d, J=7Hz), 6.55 (1H, d, J=7Hz), 7.08 (1H, t, J=7Hz), 7.20-7.50 (5H, m)
5) N-[(3,4-Dihydro-7-phenyl-2-naphthyl)methyl]-N-hydroxyacetamide
   - mp :: 123-125°C
   - IR (Nujol) :: 3250, 2925, 1620 cm⁻¹
   - NMR (CDCℓ₃, δ) :: 2.18 (3H, s), 2.34 (2H, t, J=8Hz), 2.88 (2H, t, J=8Hz), 4.41 (2H, s), 6.44 (1H, s), 7.18-7.60 (8H, m)
6) N-[(7-Benzyl-3,4-dihydro-2-naphthyl)methyl]-N-hydroxyacetamide
   - mp :: 114-116°C
   - IR (CHCℓ₃) :: 3250, 2910, 1620 cm⁻¹
   - NMR (CDCℓ₃, δ) :: 2.13 (3H, s), 2.23 (2H, t, J=8Hz), 2.83 (2H, t, J=8Hz), 3.93 (2H, s), 4.83 (2H, s), 6.82 (1H, s), 6.87 (1H, s), 6.98 (2H, d, J=6Hz), 7.17-7.31 (5H, m)
7) N-[[8-Benzyloxy-3-(2H-1-benzopyranyl)]methyl]-N-hydroxyacetamide
   - mp :: 127-130°C
   - IR (Nujol) :: 3150, 1600, 1580 cm⁻¹
   - NMR (CDCℓ₃, δ) :: 2.12 (3H, s), 4.28 (2H, s), 4.73 (2H, s), 5.13 (2H, s), 6.37 (1H, s), 6.63 (1H, d, J=5Hz), 6.75-6.83 (2H, m), 7.32-7.42 (5H, m), 7.78 (1H, br.s)
8) N-[[8-Benzyloxy-3-(2H-1-benzothiopyranyl)]methyl]-N-hydroxyacetamide
   - mp :: 103-105°C
   - IR (CHCℓ₃) :: 3250, 3000, 2900, 1635, 1565 cm⁻¹
   - NMR (CDCℓ₃, δ) :: 2.20 (3H, s), 3.39 (2H, s), 4.40 (2H, s), 5.14 (2H, s), 6.40 (1H, s), 6.25 (1H, d, J=7Hz), 6.29 (1H, d, J=7Hz), 7.00 (1H, t, J=7Hz), 7.25-7.55 (5H, m)
9) N-[[7-Benzyloxy-2-(1H-indenyl)]methyl]-N-hydroxyacetamide
   - mp :: 109-111°C
   - IR (Nujol) :: 3100, 1600, 1260 cm⁻¹
   - NMR (CDCℓ₃, δ) :: 2.17 (3H, s), 3.42 (2H, s), 4.62 (2H, s), 5.13 (2H, s), 6.72 (1H, s), 6.81 (1H, t, J=8Hz), 6.90-7.48 (7H, m)
10) N-[[2-Benzyloxy-6,7-dihydro-8-(5H-benzocycloheptenyl)]methyl)-N-hydroxyacetamide
   - mp :: 141-142.5°C
   - IR (CHCℓ₃) :: 3250, 3000, 2940, 1630, 1605 cm⁻¹
   - NMR (CDCℓ₃, δ) :: 1.80-2.10 (2H, m), 2.15 (3H, s), 2.24 (2H, t, J=7Hz), 2.72 (2H, t, J=7Hz), 4.33 (2H, s), 5.03 (2H, s), 6.36 (1H, s), 6.65-6.90 (2H, m), 7.00 (1H, d, J=7Hz), 7.25-7.50 (5H, m)
11) N-Hydroxy-N-[[8-phenoxy-3-(2H-1-benzopyranyl)]methyl]acetamide
   - mp :: 142.5-143.5°C
   - IR (CHCl₃) :: 3220, 1660, 1600, 1578, 1490, 1475, 1270, 1250, 1200 cm⁻¹
   - NMR (CDCl₃, δ) :: 2.15 (3H, s), 4.28 (2H, s), 4.70 (2H, br.s), 6.51 (1H, s), 6.80-6.90 (3H, m), 6.90-7.10 (3H, m), 7.25-7.47 (2H, m)
12) N-[(3,4-Dihydro-7-phenylthio-2-naphthyl)methyl]-N-hydroxyacetamide
   - mp :: 112-114°C
   - IR (CHCl₃) :: 3230, 3000, 2930, 1620, 1475, 1435, 1420, 1395 cm⁻¹
   - NMR (CDCl₃, δ) :: 2.15 (3H, s), 2.28 (2H, t, J=7Hz), 2.84 (2H, t, J=7Hz), 4.33 (2H, s), 6.30 (1H, s), 7.00-7.10 (2H, m), 7.10-7.40 (6H, m)
13) N-Hydroxy-N-[[7-phenoxy-2-(1H-indenyl)]methyl]acetamide
   - mp :: 100-101°C
   - IR (CHCl₃) :: 3260, 3000, 1620, 1600, 1575, 1490, 1470, 1240 cm⁻¹
   - NMR (CDCl₃, δ) :: 2.15 (3H, s), 3.33 (2H, s), 4.63 (2H, s), 6.70-6.85 (2H, m), 6.90-7.40 (7H, m)

### Example 3

To a solution of N-hydroxy-(3,4-dihydro-5-phenoxy-2-naphthyl)methylamine (267 mg) in methylene chloride (10 ml) was added dropwise methyl chloroformate (520 mg) at 0°C. After stirring for half an hour at 0°C, the mixture was quenched with ice water. The separated organic layer was washed with brine, dried, and concentrated in vacuo to give an oil, which was purified by column chromatography on silica gel (elution by n-hexane/ethyl acetate = 2/1) to yield N-hydroxy-N-methoxycarbonyl-(3,4-dihydro-5-phenoxy-2-naphthyl)methylamine (200 mg) as crystals.
- mp :: 109-110°C
- IR (Nujol) :: 3200, 1640, 1570, 1490, 1250 cm⁻¹
- NMR (CDCl₃, δ) :: 2.25 (2H, t, J=8Hz), 2.81 (2H, t, J=8Hz), 3.81 (3H, s), 4.32 (2H, s), 6.47 (1H, s), 6.80-6.97 (4H, m), 7.01-7.20 (2H, m), 7.32-7.38 (2H, m)
The following compound (Example 4) was obtained according to a similar manner to that of Example 3.

### Example 4

### N-[(3,4-Dihydro-5-phenoxy-2-naphthyl)methyl]-N-hydroxyisobutyrylamide

- mp :: 122-124°C
- IR (Nujol) :: 3150, 1600, 1490, 1340, 1240 cm⁻¹
- NMR (CDCℓ₃, δ) :: 1.10 (3H, s), 1.22 (3H, s), 2.22 (2H, t, J=8Hz), 2.71 (1H, m), 2.82 (2H, t, J=8Hz), 4.40 (2H, s), 6.40 (1H, s), 6.78-6.91 (4H, m), 7.00-7.18 (2H, m), 7.27-7.32 (2H, m), 8.52 (1H, br.s)

### Example 5

To a solution of N-hydroxy-(3,4-dihydro-5-phenoxy-2-naphthyl)methylamine (300 mg) in methylene chloride (10 ml) was added dropwise ethyl isocyanate (87 mg) at 0°C. After stirring for half an hour at 0°C, the mixture was quenched with ice water. The separated organic layer was washed with brine, dried, and concentrated in vacuo to give a solid. The solid was crystallized from a mixture of n-hexane and ethyl acetate to yield N-[(3,4-dihydro-5-phenoxy-2-naphthyl)methyl]-N-hydroxy-N'-ethylurea.
- mp :: 137-138°C
- IR (Nujol) :: 3400, 3150, 1640, 1495, 1240 cm⁻¹
- NMR (CDCℓ₃, δ) :: 1.12 (3H, t, J=8Hz), 2.22 (2H, t, J=8Hz), 2.78 (2H, t, J=8Hz), 3.25 (2H, q, J=8Hz), 4.22 (2H, s), 5.98 (1H, br.s), 6.40 (1H, s), 6.45 (1H, s), 6.77-6.90 (4H, m), 7.00-7.12 (2H, m), 7.22-7.32 (2H, m)
The following compounds (Examples 6-1) and 6-3)) were obtained according to a similar manner to that of Example 5.

### Example 6

1) N-[(3,4-Dihydro-5-phenoxy-2-naphthyl)methyl]-N-hydroxyurea
   - mp :: 153-154°C
   - IR (Nujol) :: 3500, 3200, 1640, 1570, 1490, 1340 cm⁻¹
   - NMR (CDCℓ₃, δ) :: 2.27 (2H, t, J=8Hz), 2.81 (2H, t, J=8Hz), 4.23 (2H, s), 5.36 (2H, s), 6.42 (1H, s), 6.73-7.15 (6H, m), 7.22-7.35 (2H, m)
2) N-[(3,4-Dihydro-5-phenoxy-2-naphthyl)methyl]-N-hydroxy-N'-methylthiourea
   - mp :: 153-155°C
   - IR (Nujol) :: 3360, 3050, 1540, 1330, 1250 cm⁻¹
   - NMR (CDCℓ₃, δ) :: 2.30 (2H, t, J=8Hz), 2.80 (2H, t, J=8Hz), 3.14 (3H, d, J=5Hz), 4.92 (2H, s), 6.41 (1H, s), 6.78-6.92 (4H, m), 6.99-7.14 (3H, m), 7.25-7.32 (2H, m)
3) N-[(3,4-Dihydro-5-phenoxy-2-naphthyl)methyl]-N-(tetrahydropyran-2-yloxy)-N'-ethylurea
   oil
   - IR (Neat) :: 3400, 1670, 1500, 1370, 1310, 1030, 900, 750 cm⁻¹
   - NMR (CDCl₃, δ) :: 1.19 (3H, t, J=8Hz), 1.42-1.58 (4H, m), 1.73-1.82 (2H, m), 2.20-2.43 (2H, m), 2.60-2.95 (2H, m), 3.22-3.77 (2H, m), 3.43-3.53 (1H, m), 3.93-4.03 (1H, m), 4.05 (1H, d, J=15Hz), 4.51 (1H, d, J=15Hz), 4.70-4.75 (1H, m), 6.30 (1H, t, J=5Hz), 6.47 (1H, s), 6.78-6.92 (4H, m), 7.00-7.15 (2H, m), 7.27-7.83 (2H, m)

### Example 7

To a solution of N-[(3,4-dihydro-5-phenoxy-2-naphthyl)methyl]-N-(tetrahydropyran-2-yloxy)-N'-ethylurea (30.515 g) in methanol (300 ml) was added concentrated hydrochloric acid (36.01 ml) at 0°C. After stirring at 0°C for 10 minutes, the mixture was stirred at ambient temperature for 3.5 hours. The mixture was poured into ice water (1 ℓ) and extracted with diethyl ether. The extract was washed with brine, dried, and concentrated in vacuo to give N-[(3,4-dihydro-5-phenoxy-2-naphthyl)methyl]-N-hydroxy-N'-ethylurea (23.22 g).
- mp :: 137-138°C
- IR (Nujol) :: 3400, 3150, 1640, 1495, 1240 cm⁻¹
- NMR (CDCl₃, δ) :: 1.12 (3H, t, J=8Hz), 2.22 (2H, t, J=8Hz), 2.78 (2H, t, J=8Hz), 3.25 (2H, q, J=8Hz), 4.22 (2H, s), 5.98 (1H, br.s), 6.40 (1H, s), 6.45 (1H, s), 6.77-6.90 (4H, m), 7.00-7.12 (2H, m), 7.22-7.32 (2H, m)

## Claims (Claims for the following Contracting State(s): AT, BE, CH, DE, DK, FR, GB, IT, LI, LU, NL, SE)

1. A compound of the formula : 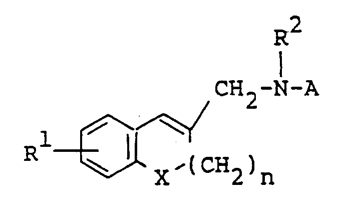 wherein
R¹ is aryl, aryloxy, arylthio, ar(lower)alkyl or ar(lower)alkoxy,
R² is acyl,
A is hydroxy or protected hydroxy,
X is -CH₂-, -O- or -S-, and
n is an integer of 0 to 2,
with proviso that X is -CH₂- when n is 0,
and its pharmaceutically acceptable salt.

2. A compound according to claim 1,
wherein
R¹ is aryloxy,
R² is lower alkylcarbamoyl,
A is hydroxy,
X is -CH₂-, and
n is 1.

3. A compound according to claim 2,
wherein
R¹ is phenoxy and
R² is ethylcarbamoyl.

4. A compound of claim 3, which is N-[3,4-dihydro-5-phenoxy-2-naphthyl)methyl]-N-hydroxy-N'-ethylurea.

5. A process for preparing a compound of the formula : 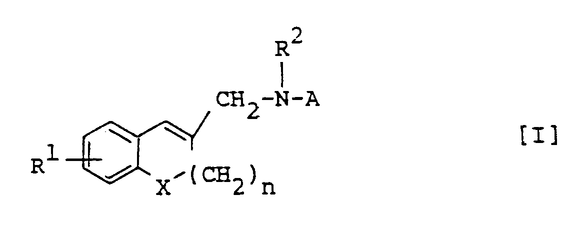 wherein
R¹ is aryl, aryloxy, arylthio, ar(lower)alkyl or ar(lower)alkoxy,
R² is acyl,
A is hydroxy or protected hydroxy,
X is -CH₂-, -O- or -S-, and
n is an integer of 0 to 2,
with proviso that X is -CH₂- when n is 0,
or its salt which comprises
reacting a compound of the formula : 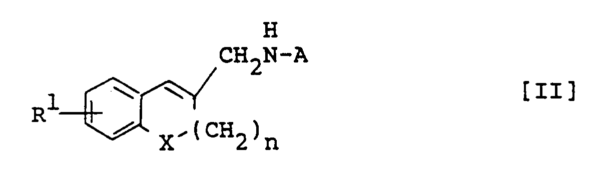 wherein R¹, A, X and n are each as defined above, or its salt with an acylating agent.

6. A process for preparing a compound of the formula : 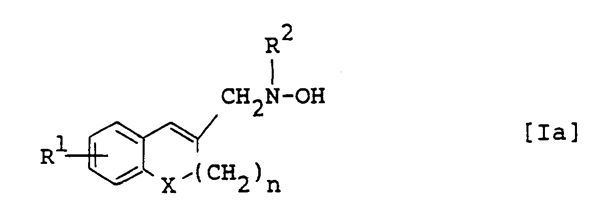 wherein
R¹ is aryl, aryloxy, arylthio, ar(lower)alkyl or ar(lower)alkoxy,
R² is acyl,
X is -CH₂-, -O- or -S-, and
n is an integer of 0 to 2,
with proviso that X is -CH₂-when n is 0,
which comprises subjecting a compound of the formula: 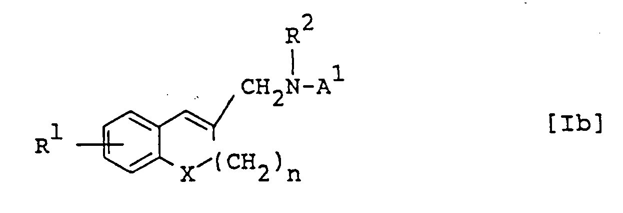 wherein
R¹, R², X and n are each as defined above, and
A¹ is protected hydroxy,
to the deprotection reaction of hydroxy-protective group.

7. A pharmaceutical composition comprising a compound of claim 1, as an active ingredient, in association with a pharmaceutically acceptable, substantially non-toxic carrier or excipient.

8. A pharmaceutical composition according to claim 7 as an inhibtitory agent against 5-lipoxygenase.

9. A pharmaceutical composition according to claim 8 as an anti-allergic agent or anti-inflammatory agent.

10. A compound of claim 1 for use as a medicament.

## Claims (Claims for the following Contracting State(s): ES)

1. A process for preparing a compound of the formula : 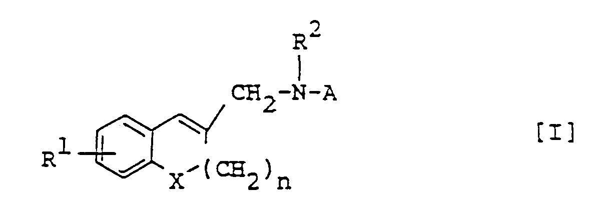 wherein
R¹ is aryl, aryloxy, arylthio, ar(lower)alkyl or ar(lower)alkoxy,
R² is acyl,
A is hydroxy or protected hydroxy,
X is -CH₂-, -O- or -S-, and
n is an integer of 0 to 2,
with proviso that X is -CH₂- when n is 0,
or its salt which comprises
reacting a compound of the formula : 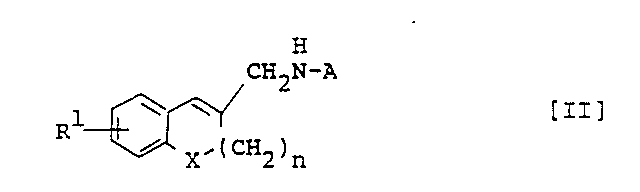 wherein R¹, A, X and n are each as defined above, or its salt with an acylating agent.

2. A process for preparing a compound of the formula : 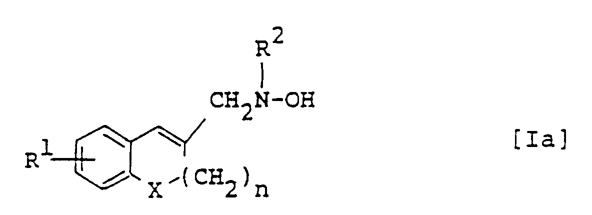 wherein
R¹ is aryl, aryloxy, arylthio, ar(lower)alkyl or ar(lower)alkoxy,
R² is acyl,
X is -CH₂-, -O- or -S-, and
n is an integer of 0 to 2,
with proviso that X is -CH₂-when n is 0,
which comprises subjecting a compound of the formula: 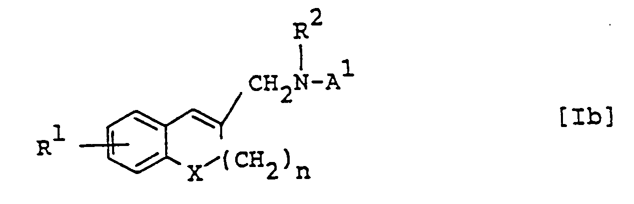 wherein
R¹, R², X and n are each as defined above, and
A¹ is protected hydroxy,
to the deprotection reaction of hydroxy-protective group.

3. A process according to claim 1,
wherein
R¹ is aryloxy,
R² is lower alkylcarbamoyl,
A is hydroxy,
X is -CH₂-, and
n is 1.

4. A process according to claim 3,
wherein
R¹ is phenoxy and
R² is ethylcarbamoyl.

5. A process of claim 4, for preparing N-[3,4-dihydro-5-phenoxy-2-naphthyl)methyl]-N-hydroxy-N'-ethylurea.

## Patentansprüche (Patentansprüche für folgende(n) Vertragsstaat(en): AT, BE, CH, DE, DK, FR, GB, IT, LI, LU, NL, SE)

1. Eine Verbindung der Formel: 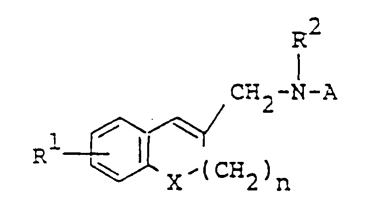 worin R¹ Aryl, Aryloxy, Arylthio, Ar(nieder)alkyl oder Ar(nieder)alkoxy ist, R² Acyl ist, A Hydroxy oder geschütztes Hydroxy ist, X -CH₂-, -O- oder -S- ist und n eine ganze Zahl von 0 bis 2 ist, mit der Maßgabe, daß x -CH₂- ist, wenn n 0 ist, und ihr pharmazeutisch verträgliches Salz.

2. Eine Verbindung nach Anspruch 1, worin R¹ Aryloxy ist, R² niederes Alkylcarbamoyl ist, A Hydroxy ist, X -CH₂- ist und n 1 ist.

3. Eine Verbindung nach Anspruch 2, worin R¹ Phenoxy und R² Ethylcarbamoyl ist.

4. Eine Verbindung nach Anspruch 3, die N-[3,4-Dihydro-5-phenoxy-2-naphthyl)methyl]-N-hydroxy-N'-ethylharnstoff ist.

5. Ein Verfahren zur Herstellung einer Verbindung der Formel: 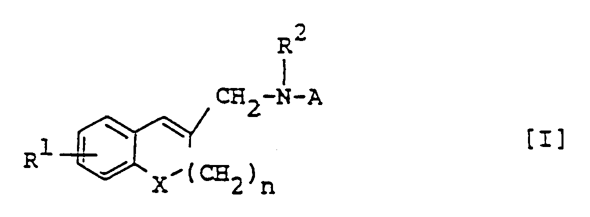 worin R¹ Aryl, Aryloxy, Arylthio, Ar(nieder)alkyl oder Ar(nieder)alkoxy ist, R² Acyl ist, A Hydroxy oder geschütztes Hydroxy ist, X -CH₂-, -O- oder -S- ist, und n eine ganze Zahl von 0 bis 2 ist, mit der Maßgabe, daß X -CH₂- ist, wenn n 0 ist oder ihres Salzes, welches das Reagieren einer Verbindung der Formel: 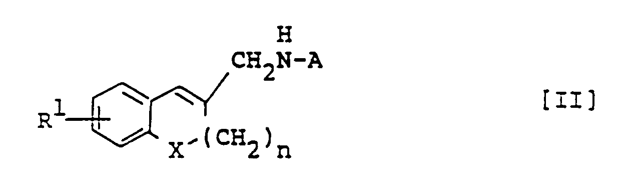 worin R¹, A, X und n jeweils wie oben definiert sind, oder ihres Salzes mit einem acylierenden Mittel umfaßt.

6. Verfahren zur Herstellung einer Verbindung der Formel: 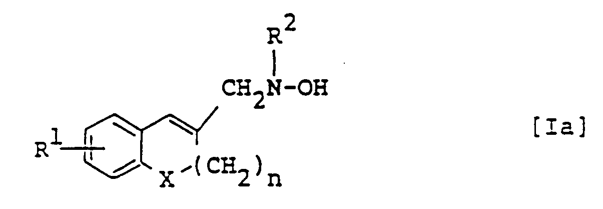 worin R¹ Aryl, Aryloxy, Arylthio, Ar(nieder)alkyl oder Ar(nieder)alkoxy ist, R² Acyl ist, X -CH₂, -O- oder -S- ist und n eine ganze Zahl von 0 bis 2 ist, mit der Maßgabe, daß X -CH₂-ist, wenn n 0 ist, das umfaßt:
Unterwerfen einer Verbindung der Formel: 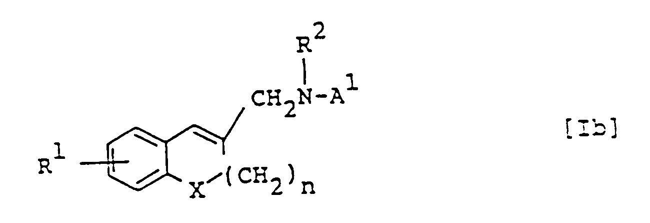 worin R¹, R², X und n jeweils wie oben definiert sind, und A¹ geschütztes Hydroxy ist, der Schutzgruppenentfernungsreaktion der Hydroxy-Schutzgruppe.

7. Eine pharmazeutische Zusammensetzung, die als einen aktiven Bestandteil eine Verbindung nach Anspruch 1 in Assoziation mit einem pharmazeutisch verträglichen, im wesentlichen nicht-toxischen Träger oder Exzipienten umfaßt.

8. Eine pharmazeutische Zusammensetzung nach Anspruch 7 als ein inhibitorisches Mittel gegen 5-Lipoxygenase.

9. Eine pharmazeutische Zusammensetzung nach Anspruch 8 als antiallergisches Mittel oder antiinflammatorisches Mittel.

10. Eine Verbindung nach Anspruch 1 zur Verwendung als Medikament.

## Patentansprüche (Patentansprüche für folgende(n) Vertragsstaat(en): ES)

1. Ein Verfahren zur Herstellung einer Verbindung der Formel: 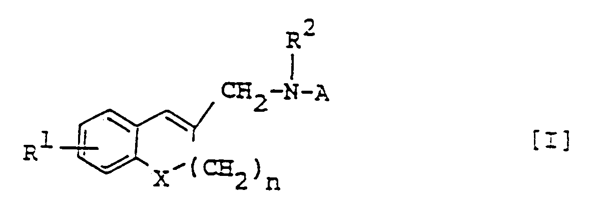 worin R¹ Aryl, Aryloxy, Arylthio, Ar(nieder)alkyl oder Ar(nieder)alkoxy ist, R² Acyl ist, A Hydroxy oder geschütztes Hydroxy ist, X -CH₂-, -O- oder -S- ist und n eine ganze Zahl von 0 bis 2 ist, mit der Maßgabe, daß X -CH₂- ist, wenn n 0 ist oder ihres Salzes, welches das Reagieren einer Verbindung der Formel: 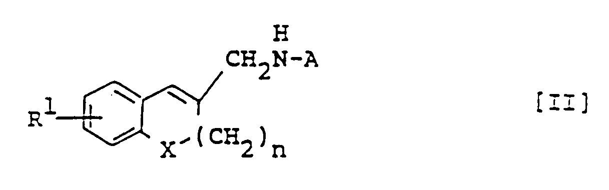 worin R¹, A, X und n jeweilt wie oben definiert sind, oder ihres Salzes mit einem acylierenden Mittel umfaßt.

2. Ein Verfahren zur Herstellung einer Verbindung der Formel: 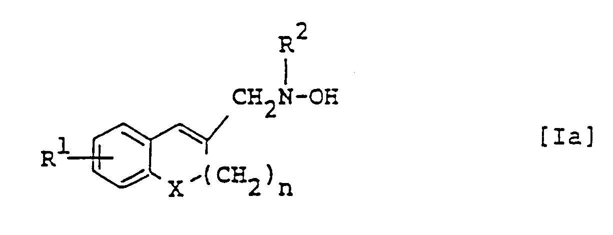 worin R¹ Aryl, Aryloxy, Arylthio, Ar(nieder)alkyl oder Ar(nieder)alkoxy ist, R² Acyl ist, X -CH₂-, -O- oder -S- ist, und n eine ganze Zahl von 0 bis 2 ist, mit der Maßgabe, daß X -CH₂-ist, wenn n 0 ist, das umfaßt:
Unterwerfen einer Verbindung der Formel: 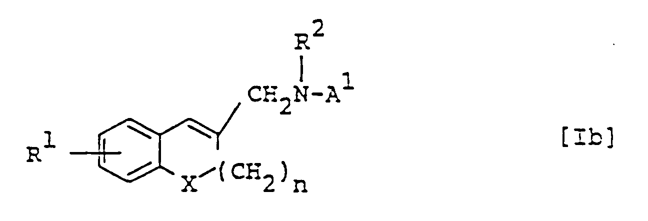 worin R¹, R², X und n jeweils wie oben definiert sind, und A¹ geschütztes Hydroxy ist, der Schutzgruppenentfernungsreaktion der Hydroxy-Schutzgruppe.

3. Ein Verfahren nach Anspruch 1, worin R¹ Aryloxy ist, R² niederes Alkylcarbamoyl ist, A Hydroxy ist, X -CH₂- ist, und n 1 ist.

4. Ein Verfahren nach Anspruch 3, worin R¹ Phenoxy ist und R² Ethylcarbamoyl ist.

5. Ein Verfahren nach Anspruch 4 zur Herstellung von N-[3,4-Dihydro-5-phenoxy-2-naphthyl)methyl]-N-hydroxy-N'-ethylharnstoff.

## Revendications (Revendications pour l'(les) Etat(s) contractant(s) suivant(s): AT, BE, CH, DE, DK, FR, GB, IT, LI, LU, NL, SE)

1. Composé de formule: 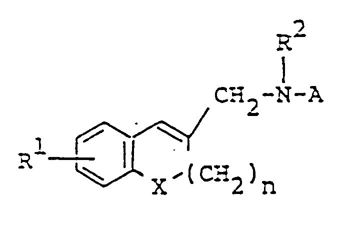 dans laquelle
R¹ est un aryle, un aryloxy, un arylthio, un aralkyle inférieur ou un aralcoxy inférieur,
R² est un acyle,
A est un hydroxy ou un hydroxy protégé,
X est -CH₂-, -O- ou -S-, et
n est un nombre entier compris entre 0 et 2,
à condition que X soit -CH₂- lorsque n est égal à 0,
et son sel pharmaceutiquement acceptable.

2. Composé selon la revendication 1,
dans lequel
R¹ est un aryloxy,
R² est un alkyl(inférieur)carbamoyle,
A est un hydroxy,
X est -CH₂-, et
n est égal à 1.

3. Composé selon la revendication 2,
dans lequel
R¹ est un phénoxy et
R² est un éthylcarbamoyle.

4. Composé selon la revendication 3, qui est la N-[3,4-dihydro-5-phénoxy-2-naphtyl)méthyl]-N-hydroxy-N'-éthylurée.

5. Procédé pour la préparation d'un composé de formule: 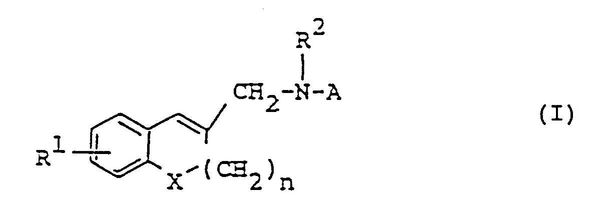 dans laquelle
R¹ est un aryle, un aryloxy, un arylthio, un aralkyle inférieur ou un aralcoxy inférieur,
R² est un acyle,
A est un hydroxy ou un hydroxy protégé,
X est -CH₂-, -O- ou -S-, et
n est un nombre entier compris entre 0 et 2,
à condition que X soit -CH₂- lorsque n est égal à 0,
ou son sel qui consiste à faire réagir un composé de formule : 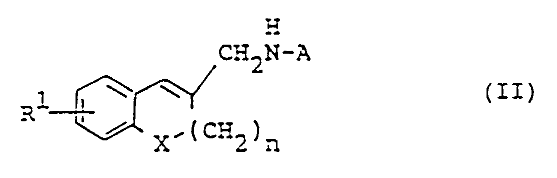 dans laquelle
R¹, A, X et n sont chacun tels que définis précédemment,
ou son sel avec un agent d'acylation.

6. Procédé pour la préparation d'un composé de formule: 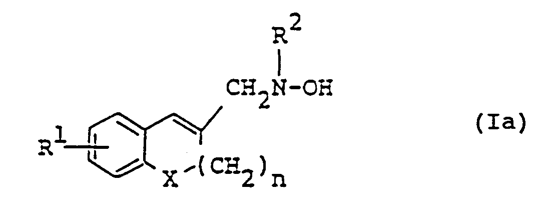 dans laquelle
R¹ est un aryle, un aryloxy, un arylthio, un aralkyle inférieur ou un aralcoxy inférieur,
R² est un acyle,
X est -CH₂-, -O- ou -S-, et
n est un nombre entier compris entre 0 et 2,
à condition que X soit -CH₂- lorsque n est égal à 0,
qui consiste à soumettre un composé de formule : 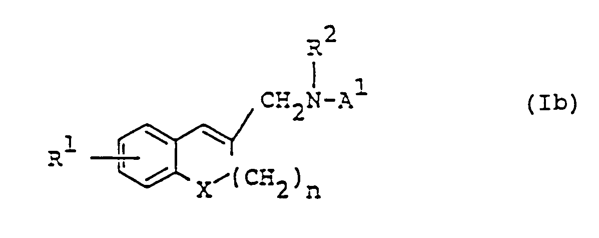 dans laquelle
R¹, R², X et n sont chacun tels que définis précédemment, et
A¹ est un hydroxy protégé,
à une réaction de déprotection du groupe protecteur de l'hydroxy.

7. Composition pharmaceutique comprenant un composé selon la revendication 1 comme ingrédient actif, en association avec un support ou un excipient pharmaceutiquement acceptable essentiellement non-toxique.

8. Composition pharmaceutique selon la revendication 7, comme agent inhibiteur de la 5-lipoxygénase.

9. Composition pharmaceutique selon la revendication 8, comme agent antiallergique ou agent anti-inflammatoire.

10. Composé selon la revendication 1, pour utilisation comme médicament.

## Revendications (Revendications pour l'(les) Etat(s) contractant(s) suivant(s): ES)

1. Procédé pour la préparation d'un composé de formule: 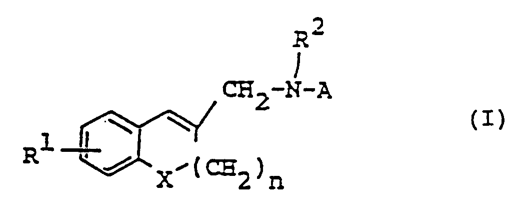 dans laquelle
R¹ est un aryle, un aryloxy, un arylthio, un aralkyle inférieur ou un aralcoxy inférieur,
R² est un acyle,
A est un hydroxy ou un hydroxy protégé,
X est -CH₂-, -O- ou -S-, et
n est un nombre entier compris entre 0 et 2,
à condition que X soit -CH₂- lorsque n est égal à 0,
ou son sel qui consiste à faire réagir un composé de formule : 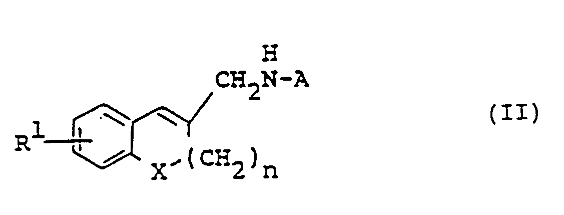 dans laquelle
R¹, A, X et n sont chacun tels que définis précédemment,
ou son sel avec un agent d'acylation.

2. Procédé pour la préparation d'un composé de formule: 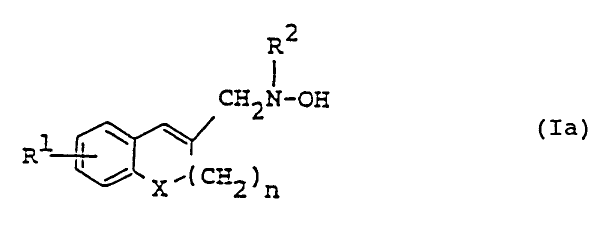 dans laquelle
R¹ est un aryle, un aryloxy, un arylthio, un aralkyle inférieur ou un aralcoxy inférieur,
R² est un acyle,
X est -CH₂-, -O- ou -S-, et
n est un nombre entier compris entre 0 et 2,
à condition que X soit -CH₂- lorsque n est égal à 0,
qui consiste à soumettre un composé de formule : 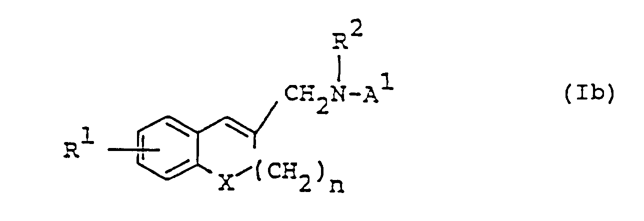 dans laquelle
R¹, R², X et n sont chacun tels que définis précédemment, et
A¹ est un hydroxy protégé,
à une réaction de déprotection du groupe protecteur de l'hydroxy.

3. Procédé selon la revendication 1,
dans lequel
R¹ est un aryloxy,
R² est un alkyl(inférieur)carbamoyle,
A est un hydroxy,
X est -CH₂-, et
n est égal à 1.

4. Procédé selon la revendication 3,
dans lequel
R¹ est un phénoxy et
R² est un éthylcarbamoyle.

5. Procédé selon la revendication 4, pour la préparation de la N-[3,4-dihydro-5-phénoxy-2-naphtyl)méthyl]-N-hydroxy-N'-éthylurée.
